(19) Europäisches Patentamt
European Patent Office
Office européen des brevets

(11) EP 1 315 700 B1

# (12) EUROPÄISCHE PATENTSCHRIFT

(45) Veröffentlichungstag und Bekanntmachung des Hinweises auf die Patenterteilung:
**02.11.2006 Patentblatt 2006/44**

(21) Anmeldenummer: **01978339.8**

(22) Anmeldetag: **05.09.2001**

(51) Int Cl.:
***C07D 211/70*** *(2006.01)*

(86) Internationale Anmeldenummer:
**PCT/EP2001/010224**

(87) Internationale Veröffentlichungsnummer:
**WO 2002/020481 (14.03.2002 Gazette 2011/02)**

(54) **SUBSTITUIERTE 4-PHENYL-1-(1-PHENYL-CYCLOHEXYL)-1,2,3,6-TETRAHYDROPYRIDINE**
SUBSTITUTED 4-PHENYL-1-(1-PHENYL-CYCLOHEXYL)-1,2,3,6-TETRAHYDROPYRIDINE
4-PHENYL-1-(1-PHENYL-CYCLOHEXYL)-1,2,3,6-TETRAHYDROPYRIDINES SUBSTITUEES

(84) Benannte Vertragsstaaten:
**AT BE CH CY DE DK ES FI FR GB GR IE IT LI LU MC NL PT SE TR**
Benannte Erstreckungsstaaten:
**LT LV RO SI**

(30) Priorität: **08.09.2000 DE 10044649**

(43) Veröffentlichungstag der Anmeldung:
**04.06.2003 Patentblatt 2003/23**

(73) Patentinhaber: **Grünenthal GmbH**
**52078 Aachen (DE)**

(72) Erfinder:
- **CHIZH, Boris**
**52072 Aachen (DE)**
- **SATTLEGGER, Michael**
**53123 Bonn (DE)**
- **HINZE, Claudia**
**52066 Aachen (DE)**
- **SUNDERMANN, Bernd**
**52066 Aachen (DE)**
- **KERWER-THOMAS, Angelika**
**52080 Aachen (DE)**
- **HENN, Gisela**
**52152 Simmerath (DE)**

(56) Entgegenhaltungen:
**WO-A-00/06545**

- **O. A. AL-DEEB: "Synthesis and Analgesic Activity of New Phenylcyclidine Derivatives" ARZNEIMITTEL FORSCHUNG, Bd. 44(II), Nr. 10, 1994, Seiten 1141-1144, XP002187672 in der Anmeldung erwähnt**

EP 1 315 700 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft substituierte 4-Phenyl-1-(1-phenyl-cyclohexyl)-1,2,3,6-tetrahydropyridine , Verfahren zu deren Herstellung, Arzneimittel enthaltend diese Verbindungen sowie die Verwendung dieser Stoffe zur Herstellung von Arzneimitteln.

Schmerz gehört zu den Basissymptomen in der Klinik. Es besteht ein weltweiter Bedarf an wirksamen Schmerztherapien. Der dringende Handlungsbedarf für eine patientengerechte und zielorientierte Behandlung chronischer und nichtchronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik oder der Grundlagenforschung zur Nociception in letzter Zeit erschienen sind. So sind beispielsweise aus J. Med. Chem. 1981, 24, 469-499 und Arzneim.-Forsch./Drug Res. 44(II), Nr.10 (1994), 1141-1144 Phencyclidin-Derivate mit analgetischer Wirkung bekannt.

In WO 00/06545 sind Verbindungen beschrieben, die eine hohe Affinität zum Nociceptin-Rezeptor ORL-1 aufweisen und sich ebenfalls zur Schmerzbekämpfung eignen.

Klassische Opioide, wie z.B. das Morphin, sind bei der Therapie starker bis sehr starker Schmerzen wirksam. Als unerwünschte Begleiterscheinungen weisen sie jedoch unter anderem Atemdepression, Erbrechen, Sedierung, Obstipation sowie eine Toleranzentwicklung auf. Sie sind außerdem bei neuropathischen oder inzidentiellen Schmerzen, wie sie insbesondere bei Tumorpatienten häufig auftreten, weniger wirksam.

Tramadolhydrochlorid - (1RS,2RS)-2-[(Dimethylamino)methyl]-1-(3-methoxyphenyl)-cyclohexanol - nimmt unter den zentral wirksamen Analgetika eine Sonderstellung ein, da dieser Wirkstoff eine starke Schmerzhemmung ohne die für Opioide bekannten Nebenwirkungen hervorruft (J. Phamacol. Exptl. Ther. 267, 33 (1993).

Kenntnisse über die physiologische Bedeutung von Ionenkanal-selektiven Substanzen sind durch die Entwicklung der patch-clamp-Technik gewonnen worden. Von besonderer Bedeutung ist der NMDA-Ionenkanal, über den ein wesentlicher Teil der Kommunikation von Synapsen abläuft. Durch diesen Ionenkanal wird der Austausch von Calciumionen zwischen einer neuronalen Zelle und ihrer Umgebung gesteuert. Die Wirkung von NMDA-Antagonisten auf den Einstrom von Calciumionen in das Zellinnere konnte mittels patch-clamp-Technik nachgewiesen werden.

Im nichtaktivierten Zustand sind die NMDA-Ionenkanäle jeweils durch einzelne Magnesiumionen verschlossen, die sich im Inneren des Kanals befinden und diesen aufgrund ihrer Größe nicht passieren können. Im aktivierten Zustand können die kleineren Calcium- und Natriumionen den Kanal passieren. Die (+)-MK801-Bindungsstelle des NMDA-Ionenkanals (ionotroper NMDA-Rezeptor) befindet sich ebenfalls im Inneren dieses Membranproteins. Substanzen mit NMDA-antagonistischer Wirkung, wie Phencyclidin (PCP), Ketamin oder MK801, besetzen diese Bindungsstelle (sogenannte "Channelblocker") und verschließen daher den betreffenden NMDA-Ionenkanal.

NMDA-Ionenkanäle spielen in vielen physiologischen und pathophysiologischen Prozessen eine wichtige Rolle, wie z.B bei der Epilepsie, der Schizophrenie, neurodegenerativen Erkrankungen, insbesondere bei Morbus Alzheimer, Morbus Huntington und Morbus Parkinson, cerebralen Ischämien und Infarkten, Psychosen bedingt durch erhöhten Aminosäurespiegel, Hirnödemen, Unterversorgungszuständen des zentralen Nervensystems, insbesondere bei Hypoxien und Anoxien, der AIDS-Demenz, der Encephalomyelitis, dem Tourette-Syndrom, der perinatalen Asphyxie und bei Tinnitus.

Die der Erfindung zugrundeliegende Aufgabe bestand in dem zur Verfügung stellen von analgetisch wirksamen Substanzen, die sich zu der Behandlung von starken Schmerzen, insbesondere zu der Behandlung von chronischen und neuropathischen Schmerzen, eignen. Sie sollten dabei sowohl μ-agonistische als auch NMDA-antagonistische Wirkung zeigen. Darüber hinaus sollten diese Wirkstoffe möglichst wenig Nebenwirkungen der Opioid-Analgetika wie z.B. Übelkeit, Erbrechen, Abhängigkeit, Atemdepression, Obstipation aufweisen.

Erfindungsgemäß wird dies durch substituierte 4-Phenyl-1-(1-phenyl-cyclohexyl)-1,2,3,6,-tetrahydropyridine der allgemeinen Formel I erreicht, wobei diese Verbindungen eine ausgeprägte analgetische Wirkung aufweisen.

Gegenstand der Erfindung sind daher substituierte 4-Phenyl-3-(1-phenyl-cyclohexyl)-1,2,3,6-tetrahydropyridine der allgemeinen Formel I,

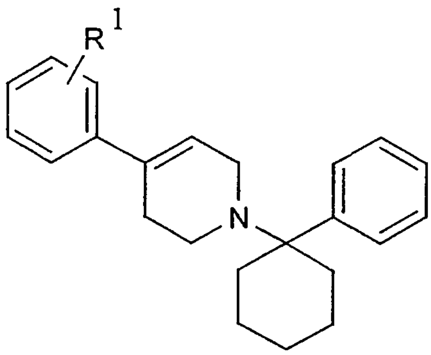

I

worin $R^1$ $C_1$-$C_5$-Alkyl (verzweigt oder unverzweigt), Halogen (außer 4-Cl), O-$C_1$-$C_5$-Alkyl (verzweigt oder unverzweigt), S-$C_1$-$C_5$-Alkyl (verzweigt oder unverzweigt) bedeutet, und/oder deren Salze von physiologisch verträglichen Säuren.

In bevorzugten Verbindungen bedeutet $R^1$ Methyl, Chlor (ausgenommen in 4-Position), Fluor, Methoxy oder Methylsulfanyl.

Besonders bevorzugt sind folgende substituierte 4-Phenyl-1-(1-phenyl-cyclohexyl)-1,2,3,6-tetrahydropyridine:

4-(2-Fluor-phenyl)-1-(2-phenyl-cyclohexyl)-1,2,3,6-tetrahydropyridin oder das entsprechende Hydrochlorid**(1)**

4-(3-Fluor-phenyl)-1-(1-phenyl-cyclohexyl)-1,2,3,6-tetrahydröpyridin oder das entsprechende Hydrochlorid **(2)**

4-(4-Fluor-phenyl)-1-(1-phenyl-cyclohexyl)-1,2,3,6-tetrahydropyridin oder das entsprechende Hydrochlorid **(3)**

4-(2-Methoxy-phenyl)-1-(1-phenyl-cyclohexyl)-1,2,3,6-tetrahydropyridin oder das entsprechende Hydrochlorid **(4)**

4-(3-Methoxy-phenyl)-1-(1-phenyl-cyclohexyl)-1,2,3,6-tetrahydropyridin oder das entsprechende Hydrochlorid **(5)**

4-(4-Methoxy-phenyl)-1-(1-phenyl-cyclohexyl)-1,2,3,6-tetrahydropyridin oder das entsprechende Hydrochlorid **(6)**

4-(2-Methyl-phenyl)-1-(1-phenyl-cyclohexyl)-1,2,3,6-tetrahydropyridin oder das entsprechende Hydrochlorid **(7)**

4-(3-Methyl-phenyl)-1-(1-phenyl-cyclohexyl)-1,2,3,6-tetrahydropyridin oder das entsprechende Hydrochlorid **(8)**

4-(4-Methyl-phenyl)-1-(1-phenyl-cyclohexyl)-1,2,3,6-tetrahydropyridin oder das entsprechende Hydrochlorid **(9)**

4-(2-Methylsulfanyl-phenyl)-1-(1-ohenyl-cyclohexyl)-1,2,3,6-tetrahydropyridin oder das entsprechende Hydrochlorid **(10)**

4-(2-Chlor-phenyl)-1-(1-phenyl-cyclohexyl)-1,2,3,6-tetrahydropyridin oder das entsprechende Hydrochlorid **(11)** .

Ein weiterer Gegenstand der Erfindung ist ein Verfahren zur Herstellung von substituierten 4-Phenyl-1-(1-phenyl-cyclohexyl)-1,2,3,6-tetrahydropyridinen der allgemeinen Formel **I,** die mit $R^1$ unterschiedlich substituiert sein können.

Die Herstellung der erfindungsgemäßen Verbindungen erfolgt in folgenden Schritten:

Aus Cylohexanon **II** und 1,4-Dioxa-8-aza-spiro [4.5]decan **III** wird das Enamin **IV** gebildet.

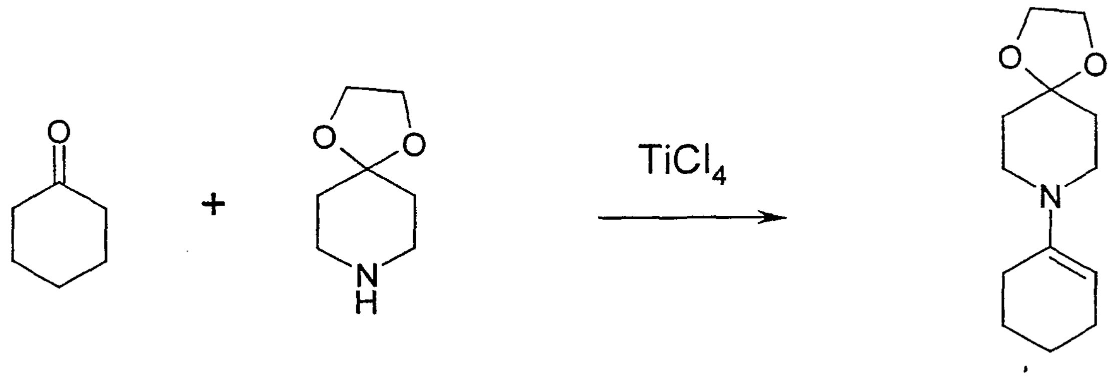


II III IV

Das Enamin **IV** wird direkt weiter mit einem Phenylmagnesium-halogenid **V** zum Amin **VI** umgesetzt:

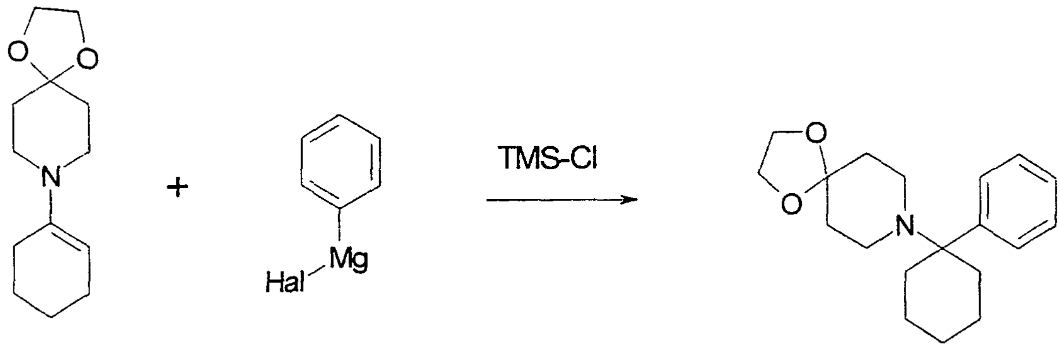


IV V VI

Das Amin **VI** wird in einem weiteren Schritt hydrolysiert und **VII** als Hydrochlorid gefällt.

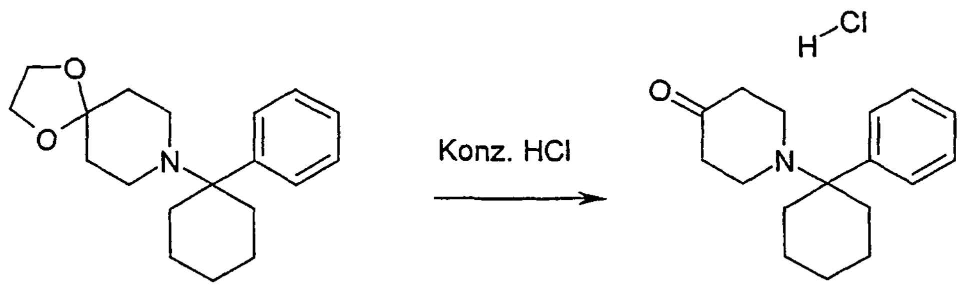


VI VII

Nach der Freisetzung des Hydrochlorids **VII** in die freie Base **VIII** erfolgt die Umsetzung mit einem Grignardreagenz **IX** zu den Verbidungen **X.**

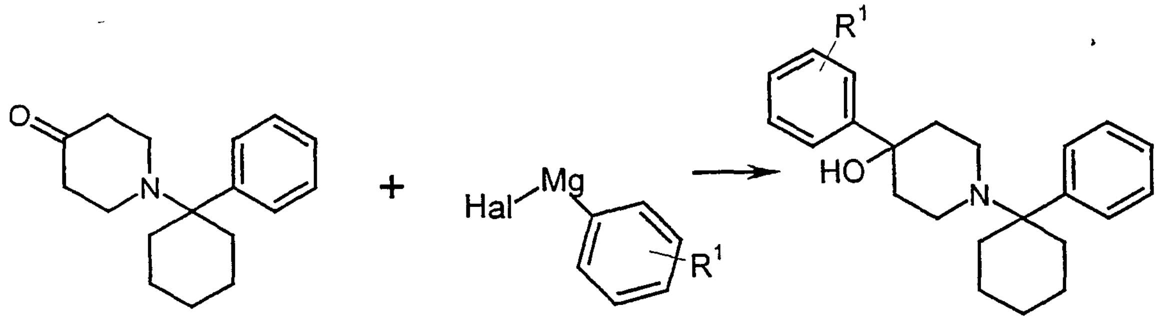

VIII IX X

Nach Fällung der Verbindungen **X** als Hydrochloride **XI** lassen sich diese weiter mit Ameisensäure zu den erfindungsgemäßen Verbindungen **I** umsetzen.

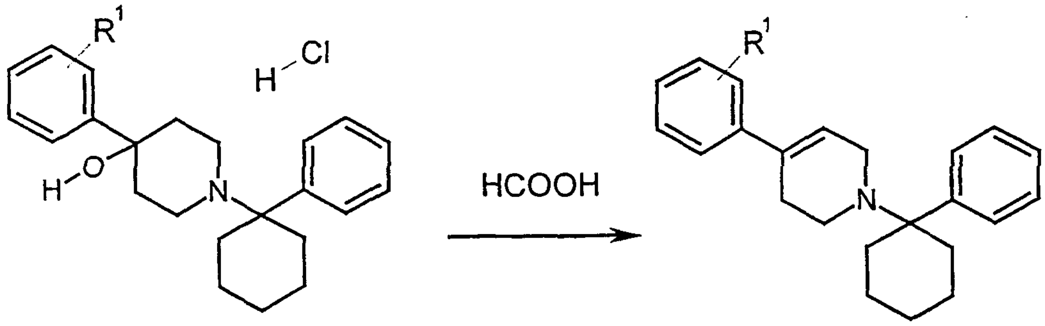


XI I

Nach einer besonders bevorzugten Variante des vorstehenden Verfahrens wird im Schritt 2, der Grignard-Reaktion, das Enamin **IV** direkt in Gegenwart von etherischer HCl weiter mit Phenylmagnesium-halogenid **V**, wobei dieses vorgelegt wird, zum Amin **VI** umgesetzt, das auf diesem Weg mit einer höheren und besser reproduzierbaren Ausbeute erhältlich ist als nach dem oben beschriebenen Verfahren.

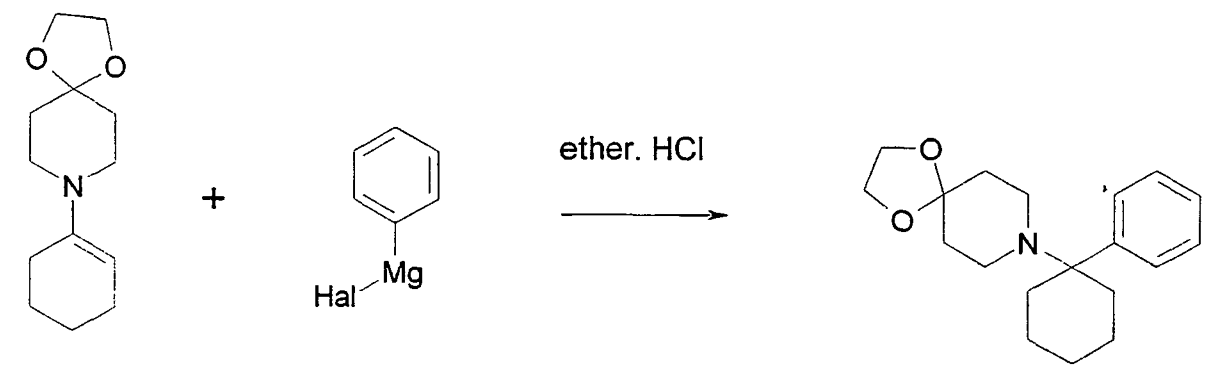


IV V VI

Eine weitere bevorzugte Verfahrensvariante wird wie folgt durchgeführt:

Aus Cylohexanon **II** und 1,4-Dioxa-8-aza-spiro [4.5]decane **III** wird in Gegenwart von Kaliumcyanid und Salzsäure das Aminonitril **XII** gebildet.

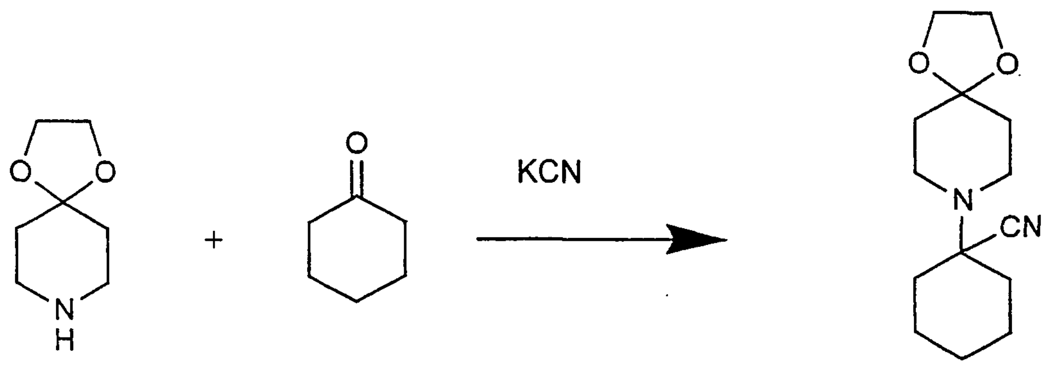


III II XII

Die Verbindung **XII** wird mit Phenyl-Grignard-Reagenzien zum Amin **VI** umgesetzt.

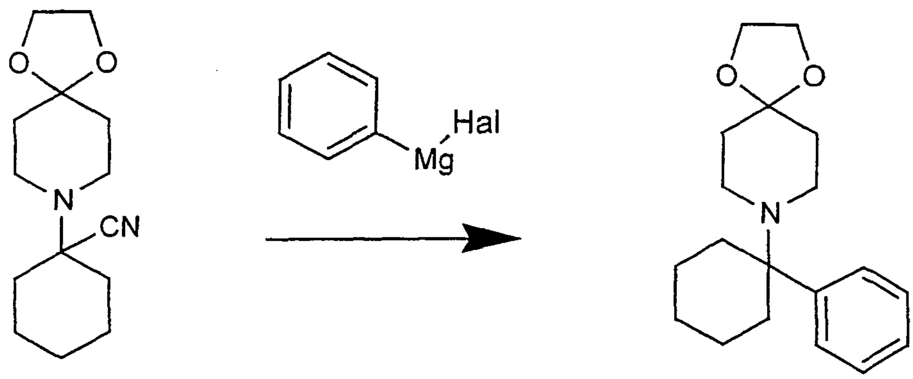


XII VI

Die nachfolgenden Schritte entsprechen denen der vorigen Wege.

Ebenfalls Gegenstand der Erfindung ist ein weiteres Verfahren zur Herstellung der substituierten 4-Phenyl-1-(1-phenyl-cyclohexyl)-1,2,3,6,-tetrahydropyridinen der allgemeinen Formel **I,** die mit $R^1$ unterschiedlich substituiert sein können, wobei zunächst aus *N-tert.* Butyloxycarbonyl-4-piperidon **XIII** und Grignard-Reagenz **IX** die Verbindung **XIV** gebildet wird:

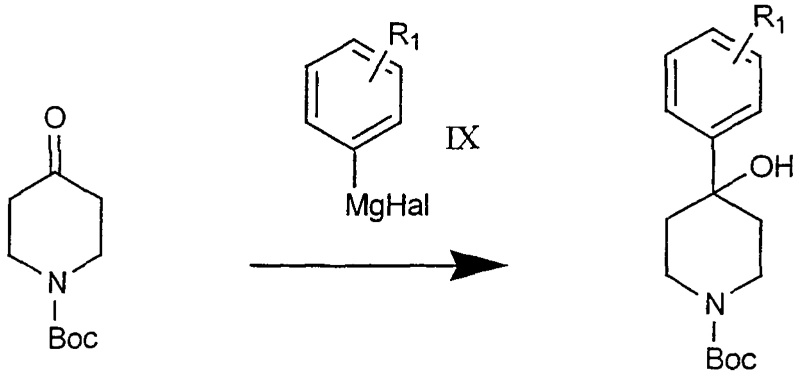

IX

XIII XIV

Aus der Verbindung **XIV** wird mit einer Säure, vorzugsweise HCl, HBr, oder HBr/Eisessig oder Ameisensäure, die tert.-Butyloxycarbonyl-Schutzgruppe abgespalten und Wasser eliminiert. Dabei entsteht Verbindung **XV** als HCl-Salz:

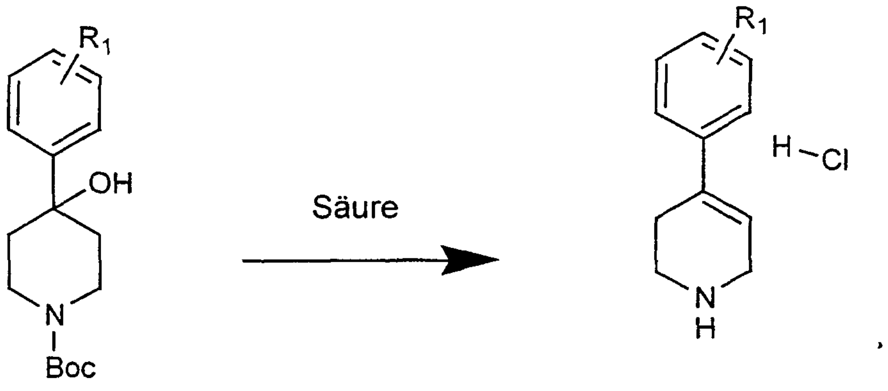


XIV XV

Verbindung **XV** wird mit Titan(IV)chlorid und Cyclohexanon **II** zu dem entsprechenden Enamin **XVI** umgesetzt:

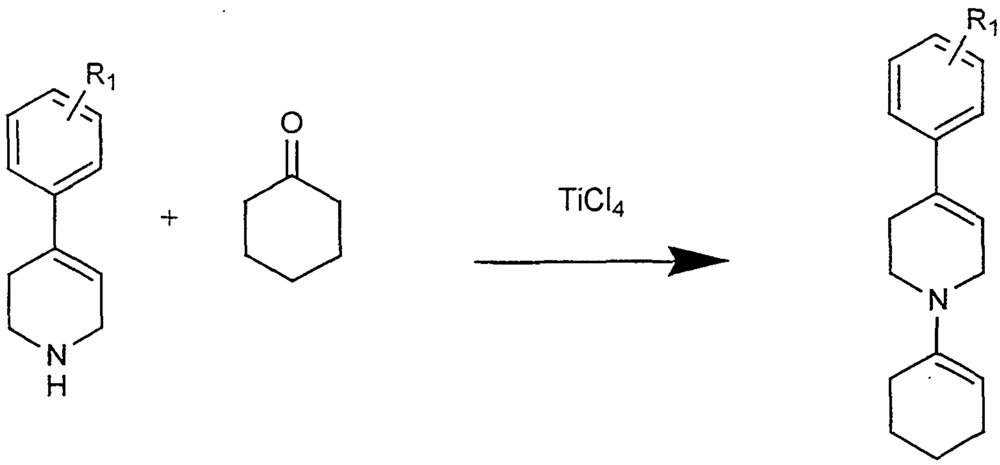


XV II XVI

Das Enamin **XVI** wird in Gegenwart von Trimethylchlorsilan oder etherischer HCl mit Phenylmagnesium-halogenid (vorzugsweise -chlorid oder -bromid) umgesetzt.

Anschließend_wird das HCl-Salz der entstandenen Verbindung der allgemeinen Formel **I** gefällt.

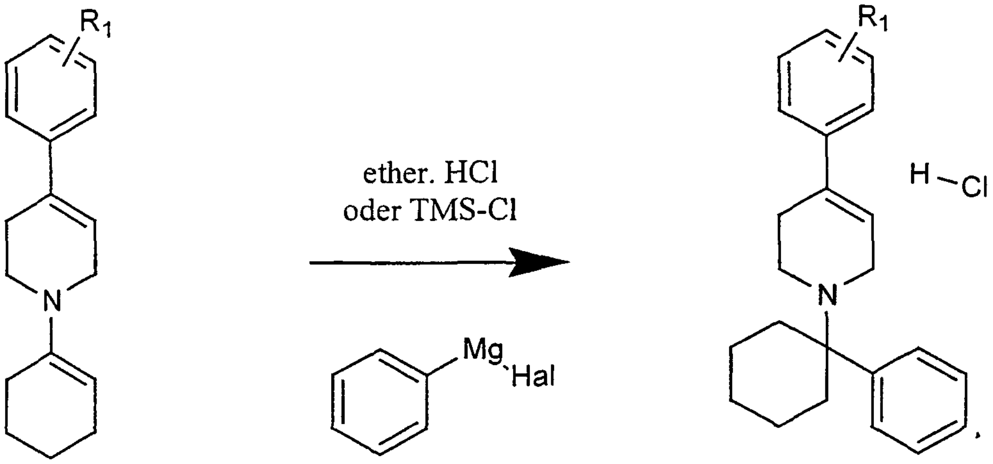


XVI

I

Die Verbindungen der allgemeinen Formel I lassen sich mit physiologisch verträglichen Säuren, beispielsweise Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure und/oder Asparaginsäure, in an sich bekannter Weise in ihre Salze überführen. Vorzugsweise wird die Salzbildung in einem Lösungsmittel, beispielsweise Diethylether, Diisopropylether, Essigsäurealkylester, Aceton und/oder 2-Butanon durchgeführt. Zur Herstellung der Hydrochloride eignet sich darüber hinaus Trimethylchlorsilan in Methylethylketon.

Die erfindungsgemäßen substituierten 4-Phenyl-1-(1-phenyl-cyclohexyl)-1,2,3,6,-tetrahydropyridine der allgemeinen Formel I sind toxikologisch unbedenklich und stellen daher geeignete pharmazeutische Wirkstoffe dar.

Ein weiterer Gegenstand der Erfindung sind daher Arzneimittel, die als Wirkstoff wenigstens ein substituiertes 4-Phenyl-1-(1-phenyl-cyclohexyl)-1,2,3,6,-tetrahydropyridin der allgemeinen Formel I und/oder deren Salze von physiologisch verträglichen Säuren enthalten

Ein weiterer Gegenstand der Erfindung ist daher auch die Verwendung von wenigstens einem substituierten 4-Phenyl-1-(1-phenyl-cyclohexyl)-1,2,3,6,-tetrahydropyridin der allgemeinen Formel I und/oder deren Salzen von physiologisch verträglichen Säuren zur Herstellung von Arzneimitteln zur Behandlung von Schmerzen, Epilepsie, und/oder Schizophrenie und/oder neurodegenerativen Erkrankungen, insbesondere Morbus Alzheimer, Morbus , Huntington oder Morbus Parkinson, und/oder cerebralen Ischämien und/oder cerebralen Infarkten und/oder Psychosen bedingt durch erhöhten Aminosäurespiegel und/oder Hirnödemen und/oder Unterversorgungszuständen des zentralen Nervensystems, insbesondere Hypoxie und/oder Anoxie, und/oder AIDS-Demenz und/oder Encephalomyelitis und/oder Tourette-Syndrom und/oder perinataler Asphyxie und/oder Tinnitus und/oder zur Prophylaxe von Schlaganfällen.

Zur Zubereitung entsprechender pharmazeutischer Formulierungen werden neben mindestens einem substituierten 4-Phenyl-1-(1-phenyl-cyclohexyl)-1,2,3,6,-tetrahydropyridin der allgemeinen Formel I Trägermaterialien, Füllstoffe, Lösungsmittel, Verdünnungsmittel, Farbstoffe und/oder Bindemittel eingesetzt. Die Auswahl der Hilfsstoffe, sowie die einzusetzenden Mengen derselben hängt davon ab, ob das Arzneimittel oral, intravenös, intraperitoneal, intradermal, intramuskulär, intranasal, buccal oder örtlich, zum Beispiel auf Infektionen an der Haut, der Schleimhäute und an den Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße Verbindungen der allgemeinen Formel I in einem Depot in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen Verbindungen der allgemeinen Formel I verzögert freisetzen.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 50 bis 500 mg/kg wenigstens eines 4-Phenyl-1-(1-phenyl-cyclohexyl)-1,2,3,6,-tetrahydropyridins der allgemeinen Formel I appliziert.

## Beispiele

### Allgemeine Bemerkungen

Die folgenden Beispiele dienen zur näheren Erläuterung der Erfindung, schränken aber den allgemeinen Erfindungsgedanken nicht ein.

Die Ausbeuten der hergestellten Verbindungen sind nicht optimiert.

Alle Schmelztemperaturen sind unkorrigiert.

Soweit nicht anders angegeben, wurde Petrolether mit dem Siedebereich von 50 bis 70 °C, benutzt. Die Angabe Ether bedeutet Diethylether.

Als stationäre Phase für die Säulenchromatographie wurde Kieselgel 60 (0,040 bis 0,063 mmm) der Firma E. Merck, Darmstadt, eingesetzt.

Die dünnschicht-chromatographischen Untersuchungen wurden mit HPLC-Fertigplatten, Kieselgel 60 F 254, der Firma E. Merck, Darmstadt, durchgeführt.

Die Mischungsverhältnisse der Laufmittel für alle chromatographischen Untersuchungen sind stets in Volumen/Volumen angegeben.

Boc bedeutet *tert.*Butyloxycarbonyl.

THF bedeutet Tetrahydrofuran.

### Beispiel 1

4-(2-Fluor-phenyl)-1-(1-phenyl-cyclohexyl)-1,2,3,6-tetrahydropyridin-Hydrochlorid **(1)**

### 1.Stufe

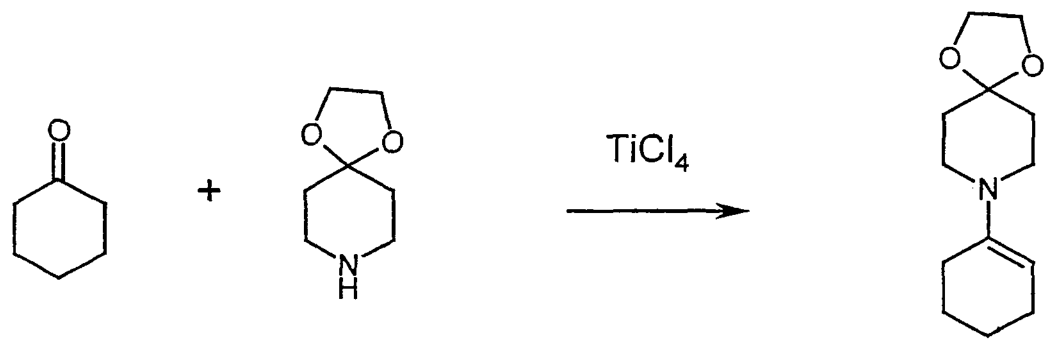


II III IV

Zur Darstellung von 8-Cyclohex-1-enyl-1,4-dioxa-8-azaspiro[4.5]decan **IV** wurden 54 ml (0,5 mol) Cylohexanon mit 200 ml (1,5 mol) 1,4-Dioxa-8-aza-spiro [4.5]decane **III** in 0,5 l Diethylether gelöst und eine halbe Stunde gerührt. Dann wurden 31 ml Titantetrachlorid in 0,5 l n-Hexan bei 0°C innerhalb von 60 min zugetropft. Nach beendeter Zugabe wurde der Ansatz langsam auf 20 °C erwärmt und 24 h nachgerührt. Der entstandene Niederschlag wurde abgesaugt und verworfen. Das Filtrat wurde eingeengt und direkt weiter umgesetzt. Die Ausbeute betrug 83 g (0,37 mol, 71 %) .

### 2. Stufe

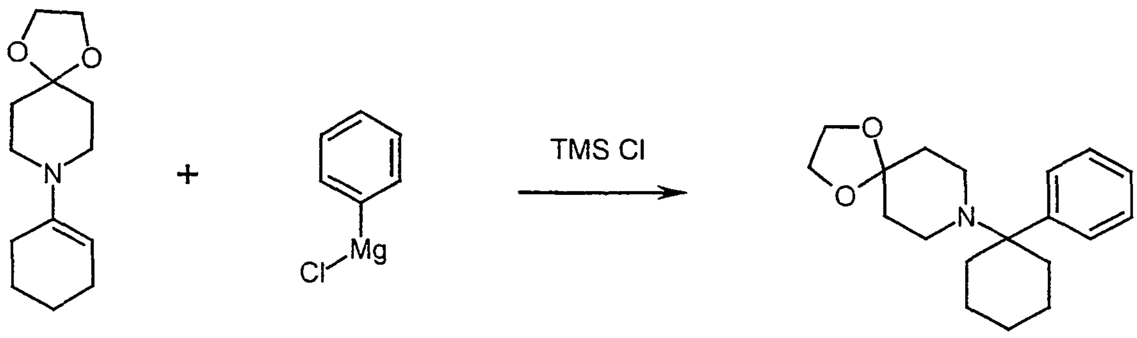


IV V VI

83 g (0,37 mol) 8-Cyclohex-1-enyl-1,4-dioxa-8-aza-spiro [4.5]decan **IV** wurden mit 200 ml (2 mol) Phenylmagnesiumchloridlösung **V** umgesetzt. Dazu wurden 52 ml Trimethylchorsilan in 0,75 l Methylenchlorid mit 2 ml Wasser vorgelegt und das Enamin **IV** zugetropft. Dann wurde unter Eisbadkühlung das Grignardreagenz zugegeben und 3 h nachgerührt. Es wurde mit 200 ml Ammoniumchloridlösung hydrolisiert und die wässrige Phase mit 0,5 l Methylenchlorid extrahiert. Das Produkt **VI** wurde säulenchromatographisch auf Kieselgel mit Diisopropylether gereinigt. Die Ausbeute betrug 36 g (0,12 mol, 32 %).

**3. Stufe**

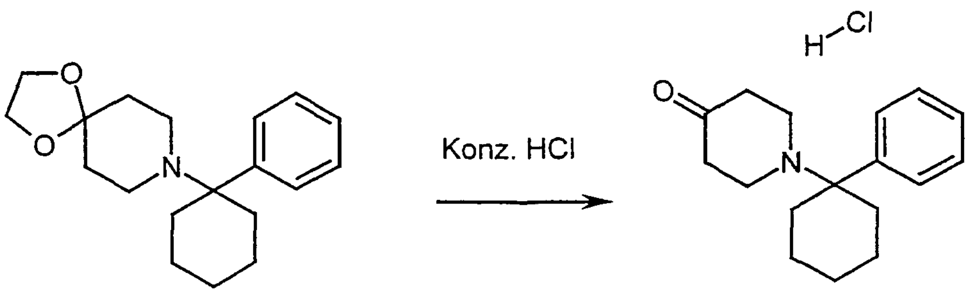


VI VII

Das Amin **VI** wurde in einem weiteren Schritt hydrolisiert und als Hydrochlorid **VII** gefällt. Dafür wurden 36 g (0,12 mol) **VI** mit 250 ml konzentrierter HCl bei 20 °C versetzt und 12 h gerührt. Es wurde mit ammoniakalischer Lösung alkalisiert und mit Diethylether extrahiert. Die freie Base wurde mit Trimethylchlorsilan als Hydrochlorid gefällt. Die Ausbeute betrug 21 g (0,072 mol, 60 %).

**4.Stufe**

Nach der Freisetzung des Hydrochlorides **VII** in die freie Base **VIII,** erfolgte die Umsetzung mit 2-Fluorphenylmagnesiumbromid **IX** zu **X** und nach Fällung als Hydrochlorid zu **XI.**

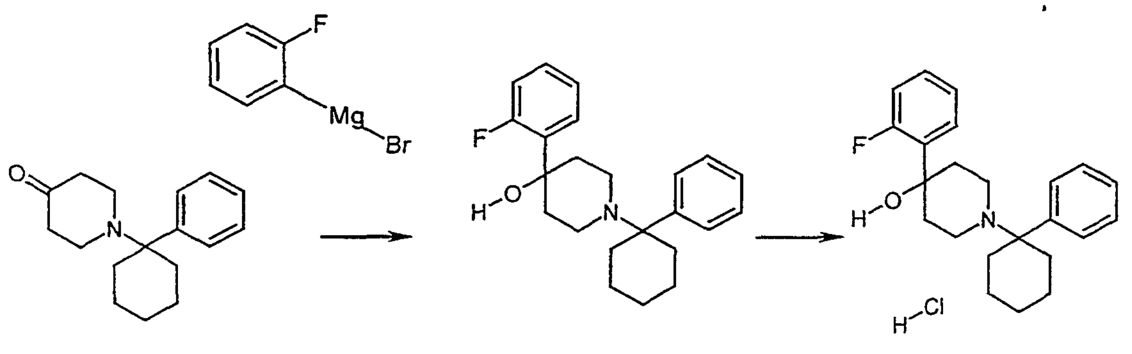

VIII IX X XI

**5.Stufe**

Das Hydrochlorid **XI** wurde 24 h in Ameisensäure als Lösungsmittel und Reagenz bei 50°C erhitzt. Anschließend wurde die Ameisensäure im Vakuum entfernt und der Rückstand in Ether gerührt. Nach Extraktion mit Ether im Alkalischen und Fällung als Hydrochlorid mit HCl/Ether in Aceton wurde **1** als farbloser Feststoff erhalten.

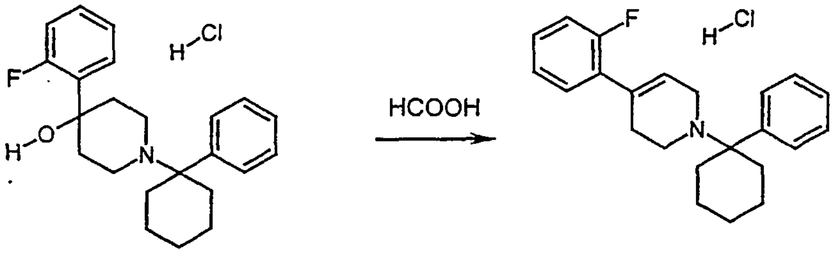


XI 1

Es wurden insgesamt 175 mg gebildet. Der Schmelzpunkt der Verbindung **1** lag bei 186°C.

**Beispiel 1a**

Die Darstellung der Verbindung **1** erfolgte wie in Beispiel 1, wobei die 2. Stufe wie folgt abgeändert war:

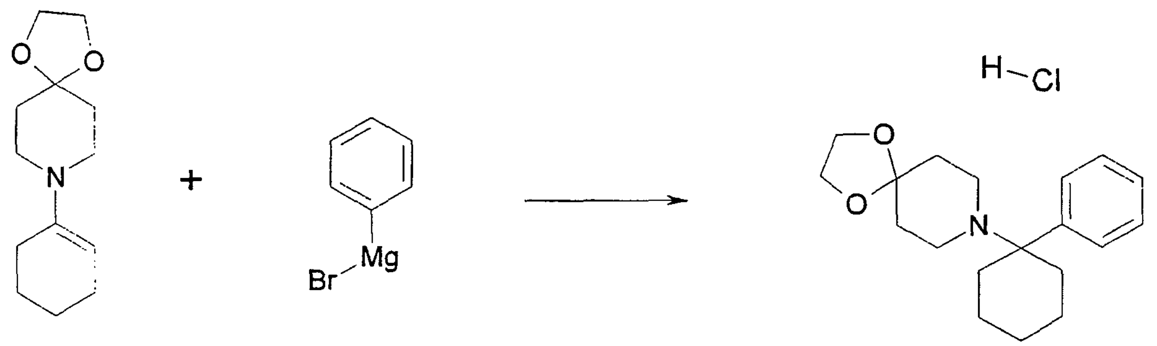

IV V VI

86,3 g (0,386 mol) 8-Cyclohex-1-enyl-1,4-dioxa-8-azaspiro[4.5]decan **IV** wurden in Dichlormethan vorgelegt und auf -10°C bis -15°C gekühlt. Zu der Lösung wurden 100 ml 20-prozentige etherische HCl getropft (pH 1). Diese Lösung wurde während 5 Stunden zu 580 ml einer käuflichen Lösung von Phenylmagnesiumbromid (1M in THF; entspricht 0,58 mol) getropft. Bei Raumtemperatur wurde über Nacht gerührt. Dann wurde mit etwa 200 ml Ammoniumchloridlösung hydrolysiert und mit Dichlormethan extrahiert. Die organische Phase wurde mit Natriumsulfat getrocknet und im Vakuum zur Trockene eingeengt. Zur Fällung des Hydrochlorides wurde die Rohbase in_etwa 1 l Diethylether gelöst und bei 0 °C mit 60 ml etherischer HCl (pH 2-3) versetzt. Die Ausbeute betrug 99,6 g (76 %).

**Beispiel 1b**

Nach dem zweiten erfindungsgemäßen Verfahren erfolgte die Darstellung der Verbindung 1 wie folgt:

1. Stufe

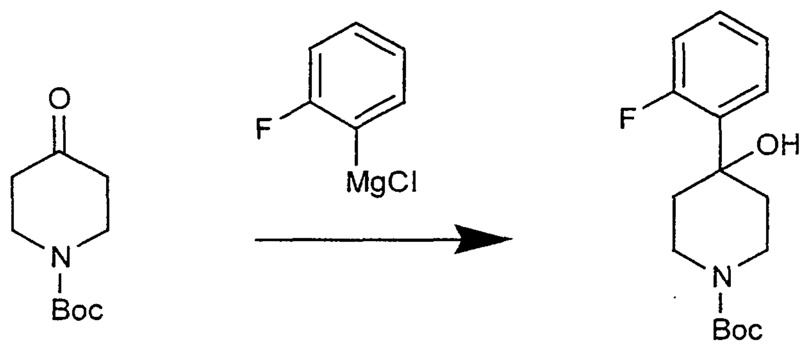

XIII XIV

Zu 27,6 ml einer käuflichen Lösung von Isopropylmagnesiumchlorid (2 M in THF) wurde bei 0 °C eine Lösung von 11,14 g 2-Fluoriodbenzol in 25 ml THF getropft. Nach 15 Minuten wurde eine Lösung von 10 g (50,2 mmol) Boc-Piperidon **XIII** in 25 ml THF zugegeben. Die Lösung wurde über Nacht bei Raumtemperatur gerührt. Dann wurde mit etwa 100 ml Ammoniumchloridlösung hydrolysiert und mit ’ Diethylether extrahiert. Die organische Phase wurde mit Natriumsulfat getrocknet und im Vakuum zur Trockene eingeengt. Die Rohausbeute betrug 15,8 g (quantitativ). Die Verbindung **XIV** wurde roh weiter umgesetzt und zwar einmal mit HCl sowie einmal mit HBr:

2. Stufe

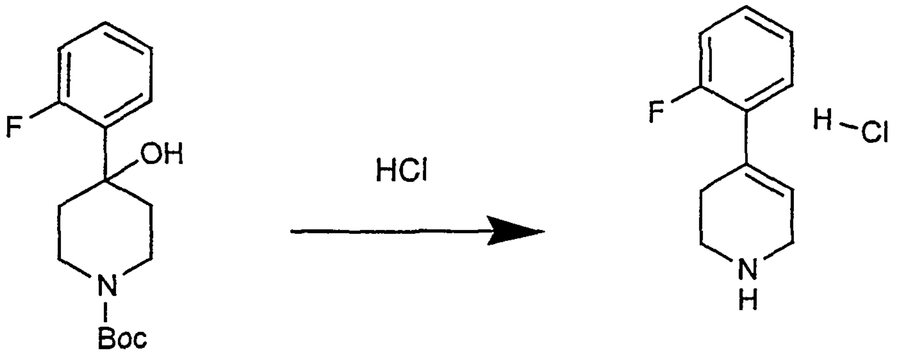


XIV XV

14,2 g (48,08 mmol) der Verbindung **XIV** wurden in 32-prozentiger HCl gelöst und über Nacht bei Raumtemperatur gerührt. Mit Diethylether wurde zunächst gegen die saure, dann gegen die mit Ammoniak alkalisch gestellte Wasserphase extrahiert. Die organische Phase wurde über Natriumsulfat getrocknet und das Lösungsmittel im Vakuum abdestilliert. Das Rohprodukt wurde in Aceton-Diethylether-Mischung gelöst und das Hydrochlorid **XV** mit etherischer HCl-Lösung gefällt.
Die Ausbeute der Reaktion betrug 3,0 g (29 %).

2. Stufe

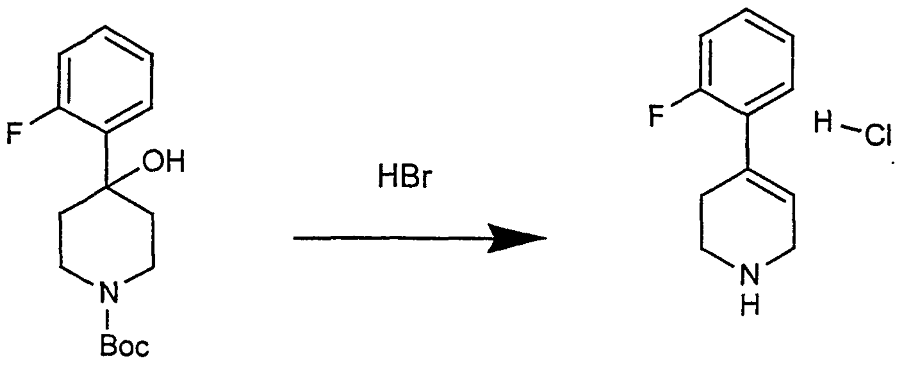


XIV XV

10,6 g (35,89 mmol) der Verbindung **XIV** wurden in etwa 100 ml 32-prozentiger Bromwasserstoffsäure suspendiert und über Nacht bei_Raumtemperatur gerührt. Nach Einengen im Vakuum wurde zunächst sauer, dann basisch gegen Diethylether extrahiert. Die organische Phase wurde über Natriumsulfat getrocknet und das Lösungsmittel im Vakuum abdestilliert. Das Rohprodukt wurde in Aceton-Diethylether-Mischung gelöst und das Hydrochlorid **XV** mit etherischer HCl ausgefällt. Die Ausbeute der Reaktion betrug 3,9 g (51 %).
Die weitere Umsetzung des Hydrochlorids **XV** erfolgte wie weiter oben für dieses Verfahren beschrieben.

**Beispiel 2**

4-(3-Fluor-phenyl)-1-(1-phenyl-cyclohexyl)-1,2,3,6-tetrahydropyridin-Hydrochlorid **(2)**

Die Darstellung der Verbindung **2** erfolgte analog der Verbindung **1.** In der vierten Stufe wurde 3-Fluorphenylmagnesiumbromid anstelle von 2-Fluorphenylmagnesiumbromid verwendet.

Dabei wurden 142 mg der Verbindung **2** erhalten. Der Zersetzungspunkt lag bei 165°C.

**Beispiel 3**

4-(4-Fluor-phenyl)-1-(1-phenyl-cyclohexyl)-1,2,3,6-tetrahydropyridin-Hydrochlorid **(3)**

Die Darstellung der Verbindung **3** erfolgte analog der Verbindung **1.** In der vierten Stufe wurde 4-Fluorphenylmagnesiumbromid anstelle von 2-Fluorphenylmagnesiumbromid verwendet.

Dabei wurden 300 mg der Verbindung **3** erhalten. Der Zersetzungspunkt lag bei 170°C.

**Beispiel 4**

4-(2-Methoxy-phenyl)-1-(1-Phenyl-cyclohexyl)-1,2,3,6-tetrahydropyridin-Hydrochlorid **(4)**

Die Darstellung der Verbindung **4** erfolgte analog der Verbindung **1.** In der vierten Stufe wurde 2-Methoxyphenylmagnesiumbromid anstelle von 2-Fluorphenylmagnesiumbromid verwendet.
Dabei wurden 168 mg der Verbindung **4** erhalten. Der Schmelzpunkt lag bei 242°C.

**Beispiel 5**

4-(3-Niethoxy-phenyl)-1-(1-Phenyl-cyclohexyl)-1,2,3,6-tetrahydropyridin-Hydrochlorid **(5)**

Die Darstellung der Verbindung **5** erfolgte analog der Verbindung **1.** In der vierten Stufe wurde 3-Methoxyphenylmagnesiumbromid anstelle von 2-Fluorphenylmagnesiumbromid verwendet.
Dabei wurden 202 mg der Verbindung **5** erhalten. Der Schmelzpunkt lag bei 214°C.

**Beispiel 6**

4-(4-Methoxy-phenyl)-1-(1-Phenyl-cyclohexyl)-1,2,3,6-tetrahydropyridin-Hydrochlorid **(6)**

Die Darstellung der Verbindung **6** erfolgte analog der Verbindung **1**. In der vierten Stufe wurde 4-Methoxyphenylmagnesiumbromid anstelle von 2-Fluorphenylmagnesiumbromid verwendet.
Dabei wurden 168 mg der Verbindung **6** erhalten. Der Schmelzpunkt lag bei 152°C.

**Beispiel 7**

4-(2-Methyl-phenyl)-1-(1-Phenyl-cyclohexyl)-1,2,3,6-tetrahydropyridin-Hydrochlorid **(7)**

Die Darstellung der Verbindung **7** erfolgte analog der Verbindung **1.** In der vierten Stufe wurde 2-Methylphenylmagnesiumbromid anstelle von 2-Fluorphenylmagnesiumbromid verwendet.
Dabei wurden 160 mg der Verbindung **7** erhalten. Der Schmelzpunkt lag bei 199°C.

**Beispiel 8**

4-(3-Methyl-phenyl)-1-(1-Phenyl-cyclohexyl)-1,2,3,6-tetrahydropyridin-Hydrochlorid **(8)**

Die Darstellung der Verbindung **8** erfolgte analog der Verbindung **1**. In der vierten Stufe wurde 3-Methylphenylmagnesiumbromid anstelle von 2-Fluorphenylmagnesiumbromid verwendet.
Dabei wurden 197 mg der Verbindung **8** erhalten. Der Schmelzpunkt lag bei 192°C.

**Beispiel 9**

4-(4-Methyl-phenyl)-1-(1-Phenyl-cyclohexyl)-1,2,3,6-tetrahydropyridin-Hydrochlorid **(9)**

Die Darstellung der Verbindung **9** erfolgte analog der Verbindung **1.** In der vierten Stufe wurde 4-Methylphenylmagnesiumbromid anstelle von 2-Fluorphenylmagnesiumbromid verwendet.
Dabei wurden 194 mg der Verbindung **9** erhalten. Der Schmelzpunkt lag bei 169°C.

**Beispiel 10**

4-(2-Methylsulfanyl-phenyl)-1-(1-phenyl-cyclohexyl)-1,2,3,6-tetrahydropyridin-Hydrochlorid **(10)**

Die Darstellung der Verbindung **10** erfolgte analog der Verbindung **1**. In der vierten Stufe wurde 2-Methylsulfanylphenylmagnesiumbromid anstelle von 2-Fluorphenylmagnesiumbromid verwendet.
Dabei wurden 94 mg der Verbindung **10** erhalten. Der Schmelzpunkt lag bei 206°C.

**Beispiel 11**

4-(2-Chlor-phenyl)-1-(1-phenyl-cyclohexyl)-1,2,3,6-tetrahydropyridin-Hydrochlorid **(11)**

Die Darstellung der Verbindung **11** erfolgte analog der Verbindung **1.** In der vierten Stufe wurde 2-Chlorphenylmagnesiumbromid anstelle von 2-Fluorphenylmagnesiumbromid verwendet.
Dabei wurden 113 mg der Verbindung **11** erhalten. Der Schmelzpunkt lag bei 98°C.

**Vergleichsbeispiel 1**

Es wurde die Verbindung gemäß Tabelle 6 mit $R^{11}$= H und CH($Z^1$) ($Z^2$) = Benzhydryl aus WO 00/06545 (siehe Seite 41) nachsynthetisiert.

**Vergleichsbeispiel 2**

Es wurde die Verbindung gemäß Beispiel 1 aus WO 00/06545 (siehe Seite 15) nachsynthetisiert.

**Pharmakologische Untersuchungen**

Writhing-Test an der Maus

Die analgetische Wirksamkeit der erfindungsgemäßen Verbindungen im Phenylchinon-induzierten Writhing-Test, modifiziert nach I.C. Hendershot, J. Forsaith in J. Pharmacol. Exp. Ther. 125, 237-240 (1959), wurde an der Maus untersucht. Hierzu wurden männliche Mäuse mit einem Gewicht von 25 bis 30 g verwendet. Gruppen von jeweils 10 Tieren pro Substanzdosis erhielten 10 Minuten nach intravenöser Gabe der Prüfsubstanzen 0,3 ml/Maus einer 0,02 %igen wässrigen Lösung von Phenylchinon (Phenylbenzochinon, Fa. Sigma, Deisenhofen; Herstellung der Lösung unter Zusatz von 5 % Ethanol und Aufbewahrung im Wasserbad bei 45 °C) intraperitoneal appliziert. Die Tiere wurden anschließend einzeln in Beobachtungskäfige gesetzt. Mit Hilfe eines Drucktastenzählers wurde die Anzahl der Schmerz-induzierten Streckbewegungen (sogenannte Writhing-Reaktionen = Durchdrücken des Körpers mit Abstrecken der Hinterextremitäten) 5 bis 20 Minuten nach der Phenylchinon-Gabe ausgezählt. Als Kontrolle wurden Tiere mitgeführt, die nur physiologische Kochsalzlösung mit Phenylchinon erhielten.
Alle Substanzen wurden in der Standarddosis von 10 mg/kg getestet. Die prozentuale Hemmung (% Hemmung) der Writhingreaktionen durch eine Substanz wurde nach folgender Formel berechnet:

$$\%\ \text{Hemmung} = 100 - \left[\frac{\text{Writhingreaktion behandelte Tiere}}{\text{Writhingreaktion Kontrolle}} \times 100\right]$$

Alle untersuchten erfindungsgemäßen Verbindungen zeigten eine mittelstarke bis starke analgetische Wirkung.
Die Ergebnisse ausgewählter Writhing-Untersuchungen sind in der **Tabelle 1** zusammengefaßt.

**Tabelle 1:** Analgesieprüfung im Writhing-Test an der Maus

| Beispiel Nr. | % Hemmung der Writhing-Reaktionen 10 mg/kg i.v. |
|---|---|
| 1 | 72 |
| 2 | 98 |
| 3 | 100 |

**Molekularbiologische Untersuchungen:**

μ-Opiatrezeptor-Bindungsuntersuchungen

a) Bestimmung der Affinität zum μ-Opiatrezeptor an der Ratte

Die Untersuchungen zur Bestimmung der Affinität der erfindungsgemäßen Verbindungen der Formel I zum μ-Opiatrezeptor wurde an Hirnmembranhomogenaten (Homogenat von Rattenhirn ohne Cerebellum, Pons und Medulla oblongata von männlichen Wistar-Ratten) durchgeführt.

Hierzu wurde das jeweils frisch präparierte Rattenhirn unter Eiskühlung in 50 mmol/l Tris-HCl (pH 7,4) homoge nisiert und für 10 Minuten bei 5.000 g und 4°C zentrifugiert. Nach Dekantieren und Verwerfen des Überstandes, erneutem Aufnehmen und Homogenisieren des Membransedimentes in 50 mmol/l Tris-HCl (pH 7,4) wurde das Homogenat anschließend für 20 Minuten bei 20.000 g und 4°C zentrifugiert. Dieser Waschschritt wurde nochmals wiederholt. Danach wurde der Überstand dekantiert und das Membransediment in kaltem 50 mmol/l Tris-HCl , 20 % Glycerol (w/v), 0,01 % Bacitracin (w/v) (pH 7,4) homogenisiert und in Aliquoten bis zur Testung eingefroren. Für die Rezeptorbindungstestung wurden die Aliquote aufgetaut und 1:10 mit dem Bindungstest-Puffer verdünnt. Im Bindungstest wurde als Puffer ein 50 mmol/l Tris-HCL, 5 mmol/l MgCl (pH 7,4), sowie als radioaktiver Ligand 1 nmol/l tritiiertes Naloxon eingesetzt. Die Ergebnisse sind in Tabelle 2 gezeigt.

b) Bestimmung der Affinität zum humanen μ-Opiatrezeptor Die Rezeptoraffinität zum humanen μ-Opiatrezeptor wurde in einem homogenen Ansatz in Mikrotiterplatten bestimmt. Hierzu wurden Verdünnungsreihen der zu prüfenden Substanzen mit einer Rezeptormembranpräparation (15 - 40 μg Protein / 250 μl Inkubationsansatz) von CHO-K1-Zellen, welche den humanen μ-Opiatrezeptor exprimieren (RB-HOM-Rezeptormembran-Präparation von Fa. NEN, Zaventem, Belgien) in Gegenwart von 1 nmol/l des radioaktiven Liganden [$^3$H]-Diprenorphine (NET1121, Fa. NEN, Zaventem, Belgien) sowie von 1 mg WGA-SPA-Beads (Wheat germ agglutinin SPA Beads der FA. Amersham/Pharmacia, Freiburg, Deutschland) in einem Gesamtvolumen von 250 μl für 90 Minuten bei Raumtemperatur inkubiert. Als Inkubationspuffer wurden 50 mmol/l Tris-HCl_supplementiert mit 0,05 % Natriumazid und mit 0,06 % bovinem Serumalbumin verwendet. Zur Bestimmung der unspezifischen Bindung wurden zusätzlich 25 μmol/l Naloxon zugegeben. Nach Beendigung der neunzigminütigen Inkubationszeit wurden die Mikrotiterplatten für 20 Minuten bei 1000 g abzentrifugiert und die Radioaktivität in einem β-Counter (Microbeta-Trilux, Fa. PerkinElmer Wallac, Freiburg, Deutschland) vermessen. Ausgehend von der prozentualen Verdrängung des radioaktiven Liganden aus seiner Bindung zum humanen μ-Opiatrezeptor durch unterschiedliche Konzentrationen der Prüfsubstanzen wurden $IC_{50}$ Hemmkonzentrationen berechnet, die eine 50-prozentige Verdrängung des radioaktiven Liganden bewirken. Durch Umrechnung mittels der Cheng-Prusoff-Beziehung wurden $K_i$-Werte für die Prüfsubstanzen erhalten.
Die Ergebnisse sind in Tabelle 3 gezeigt.

NMDA Rezeptor-Bindungsuntersuchungen

Die Untersuchungen zur Bestimmung der NMDA antagonistischen Wirkung der jeweiligen Verbindung der allgemeinen Formel I wurde an Hirnmembranhomogenaten (Homogenat von Rattenhirn ohne Cerebellum, Pons und Medulla oblongata von männlichen Wistar-Ratten (Charles River, Sulzfeld, Deutschland)) durchgeführt.
Hierzu wurden frisch präparierte Rattengehirne nach Abtrennen von Cerebellum, Pons und Medulla oblongata in 50 mmol/l Tris/HCl (pH 7,7) mit einem Polytron-Homogenisator (Modell PT3000, Kinematika AG, Littau, Schweiz) bei 6.000 Umdrehungen pro Minute (UPM) für 1 Minute unter Eiskühlung aufgeschlossen und anschließend für 15 Minuten bei 4 °C und 60.000 g zentrifugiert. Nach Dekantieren und Verwerfen des Überstandes, erneutem Aufnehmen in 50 mmol/l Tris/HCl (pH 7,7) und Aufschluß des Membranpellets mit einem Homogenisator bei 2.000 UPM für 1 Minute wurde erneut für 15 Minuten bei 4 °C und 60.000 g zentrifugiert. Der Überstand wurde wiederum verworfen und das Membranpellet in 50 mmol/l Tris/HCl (pH 7,7) homogenisiert (2.000 UPM für 1 Minute) und aliquotiert bei -70°C eingefroren.

Für den Rezeptorbindungstest wurden jeweils Aliquote aufgetaut und anschließend für 15 Minuten bei 4 °C und 60.000 g zentrifugiert. Nach Dekantieren und Verwerfen des Überstandes wurde das Membranpellet für den Bindungstest mit Bindungstest-Puffer aufgenommen und homogenisiert (2.000 UPM für 1 Minute). Als Bindungstest-Puffer wurden 5 mmol/l Tris/HCl (pH 7,7) supplementiert mit 30 μmol/l Glycin und 100 μmol/l Glutaminsäure verwendet.

Als radioaktiv markierter Ligand wurde 1 nmol/l ($^3$H)-(+)-MK801 ((5R,10S)-(+)-5-Methyl-10,11-dihydro-5H-dibenzo(a,d)cyclohepten-5,10-imin (NET-972, NEN, Köln Deutschland) zugegeben. Der Anteil an unspezifischer Bindung wurde in Anwesenheit von 10 μmol/l nicht radioaktiv markiertem (+)-MK801 (RBI/Sigma, Deisenhofen, Deutschland) bestimmt. In weiteren Ansätzen wurden die jeweiligen Verbindungen der allgemeinen Formel I in Konzentrationsreihen zugegeben und die Verdrängung des radioaktiven Liganden aus seiner spezifischen Bindung am NMDA-Rezeptor ermittelt. Alle Ansätze wurden als Dreifachbestimmungen ausgeführt. Die Ansätze wurden jeweils für 40 Minuten bei 25 °C im Wasserbad inkubiert und anschließend zur Bestimmung des an das Hirnmembranhomogenat gebundenen radioaktiven Liganden mittels Filtration durch Glasfaserfilter (GF/B) (Typ Whatman GF/B, Hassel, München, Deutschland) geerntet. Die durch die Glasfaserfilterscheiben zurückgehaltene Radioaktivität wurde nach Zugabe von Szintillator (Szintillator "Ready Protein", Beckmann Coulter GmbH, Krefeld, Deutschland) im β-Counter (Packard TRI-CARB Liquid Szintillation Analyzer 2000CA, Packard Instrument, Meriden, CT 06450, USA) vermessen.

Die aus Dreifachansätzen resultierende prozentuale Hemmung der spezifischen Bindung des Liganden ($^3$H)-(+)-MK801 in Gegenwart von je 10 μmol/l der jeweiligen Verbindung der allgemeinen Formel I dient als Maß für die Affinität dieser Verbindung zu der (+)-MK801-Bindungsstelle des ionotropen NMDA-Rezeptors.

Aus Ansätzen mit Konzentrationsreihen dieser Verbindungen der allgemeinen Formel I wurden $IC_{50}$-Werte (Konzentration der substituierten Verbindungen mit 50 % Verdrängung des radioaktiven Liganden aus seiner spezifischen Bindung) nach dem Massenwirkungsgesetz mittels nichtlinearer Regression berechnet. Aus diesen $IC_{50}$-Werten wurden nach der Cheng-Prusoff-Gleichung (Y. Cheng, W.H. Prusoff, 1973, Biochem. Pharmacol., 22, Seiten 3099-3108) $K_i$-Werte berechnet.

Die Ergebnisse sind in Tabelle 2 und 3 gezeigt.

**Tabelle 2:** Molekularbiologische Untersuchungen

| Verbindung gemäß Beispiel Nr. | Naloxon $K_i$ (μM) | MK801 $K_i$ (μM) |
|---|---|---|
| 1 | 0,24 | 0,6 |
| 2 | 0,2 | 3,9 |
| 3 | 0,06 | 1,7 |
| 4 | 50% | 5,2 |
| 5 | 0,8 | 1,6 |
| 6 | 20% | 11,1 |
| 7 | | 5,3 |
| 8 | 0,4 | 1,4 |
| 9 | 0,3 | 4,5 |
| 11 | | 2,8 |

Die erfindungsgemäßen Verbindungen zeigen vorteilhafterweise ein sehr ausgewogenes Verhältnis von μ-agonistischer und NMDA-antagonistischer Wirkung (Abweichungen zwischen den entsprechenden Ki-Werten grundsätzlich nicht größer als Faktor 10) im Vergleich zu den Verbindungen nach WO 00/06545 und sind damit besonders gut für die Behandlung von neurophatischen Schmerzen und der oben genannten Erkrankungen geeignet.

**Tabelle 3:** Molekularbiologische Untersuchungen

| Verbindung gemäß | MK801-Bindung Ki (μM) | Human μ-OR-Bindung Ki (μM) |
|---|---|---|
| Beispiel 1 | 0,57 | 0,21 |
| Vergleichsbeispiel 1 | 63,3 | 1,27 |
| Vergleichsbeispiel 2 | 8,7 | 0,38 |

Insbesondere konnte für die Verbindung gemäß Beispiel 1 die in vivo Wirksamkeit nachgewiesen werden:

**Hemmung des Wind-up-Phänomens an der Ratte:**

NMDA-Antagonisten hemmen die bei einer repetitiven elektrischen Stimulation induzierbare erhöhte Entladungsrate von spinalen Neuronen. Dieses Phänomen ist als sogenanntes Wind-Up-Phänomen beschrieben (Chizh, B.A., and Headley, P.M. (1994) Thyrotropin-releasing hormone (TRH)-induced facilitation of spinal neurotransmission: a role for NMDA receptors. Neuropharmacology, Vol. 33, 115-121.) .
Die Verbindung gemäß Beispiel 1 wurde nach der in vorstehender Literaturstelle beschriebenen Methodik in einer Dosierung von 21,5 mg/kg i.v. untersucht. Sie hemmte das Wind-Up-Phänomen an der Ratte (70 % Hemmung) und zeigt damit eine bei einem zentral verfügbaren NMDA-Antagonismus einer Substanz zu erwartende in vivo Wirkung. Die Verbindung gemäß Vergleichsbeispiel 1 zeigte hingegen bei gleicher Dosierung keinerlei Beeinflußung des Wind-Up-Phänomens.
Die erfingungsgemäßen Verbindungen fallen unter die allgemeine Formel der Anmeldung WO 00/06545, sind aber dort an keiner Stelle expressis verbis genannt und gehören dort auch nicht zu den bevorzugten Verbindungen. Sie heben sich in ihren Eigenschaften im Vergleich zu den bereits offenbarten Verbindungen deutlich aus diesen hervor.

**Patentansprüche**

1. Substituierte 4-Phenyl-1-(1-phenyl-cyclohexyl)-1,2,3,6,-tetrahydropyridine der allgemeinen Formel I,

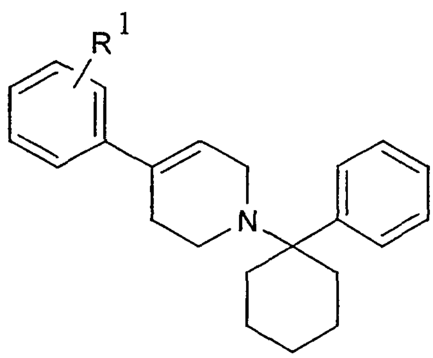

I

worin $R^1$ $C_1$-$C_5$-Alkyl (verzweigt oder unverzweigt), Halogen (ausgenommen 4-C1), O-$C_1$-$C_5$-Alkyl (verzweigt oder unverzweigt), S-$C_1$-$C_5$-Alkyl (verzweigt oder unverzweigt) bedeutet, und/oder deren Salze von physiologisch verträglichen Säuren.

2. Verbindungen gemäß Anspruch 1, **dadurch gekennzeichnet, daß** $R^1$ Methyl, Chlor (ausgenommen in 4-Position), Fluor, Methoxy oder Methylsufanyl bedeutet.

3. Verbindungen gemäß Anspruch 1:

4-(2-Fluor-phenyl)-1-(1-phenyl-cyclohexyl)-1,2,3,6-tetrahydropyridin oder das entsprechende Hydrochlorid
4-(3-Fluor-phenyl)-1-(1-phenyl-cyclohexyl)-1,2,3,6-tetrahydropyridin oder das entsprechende Hydrochlorid
4-(4-Fluor-phenyl)-1-(1-phenyl-cyclohexyl)-1,2,3,6-tetrahydropyridin oder das entsprechende Hydrochlorid
4-(2-Methoxy-phenyl)-1-(1-phenyl-cyclohexyl)-1,2,3,6-tetrahydropyridin oder das entsprechende Hydrochlorid
4-(3-Methoxy-phenyl)-1-(1-phenyl-cyclohexyl)-1,2,3,6-tetrahydropyridin oder das entsprechende Hydrochlorid
4-(4-Methoxy-phenyl)-1-(1-phenyl-cyclohexyl)-1,2,3,6-tetrahydropyridin oder das entsprechende Hydrochlorid
4-(2-Methyl-phenyl)-1-(1-phenyl-cyclohexyl)-1,2,3,6-tetrahydropyridin oder das entsprechende Hydrochlorid
4-(3-Methyl-phenyl)-1-(1-phenyl-cyclohexyl)-1,2,3,6-tetrahydropyridin oder das entsprechende Hydrochlorid
4-(4-Methyl-phenyl)-1-(1-phenyl-cyclohexyl)-1,2,3,6-tetrahydropyridin oder das entsprechende Hydrochlorid
4-(2-Methylsulfanyl-phenyl)-1-(1-phenyl-cyclohexyl)-1,2,3,6-tetrahydropyridin oder das entsprechende Hydrochlorid

4-(2-Chlor-phenyl)-1-(1-phenyl-cyclohexyl)-1,2,3,6-tetrahydropyridin oder das entsprechende Hydrochlorid.

**4.** Verfahren zur Herstellung von substituierten 4-Phenyl-1-(1-phenyl-cyclohexyl)-1,2,3,6-tetrapyridinen der allgemeinen Formel I nach Anspruch 1, wobei man Cylohexanon (Formel **II)** mit 1,4-Dioxa-8-aza-spiro [4.5]decane (Formel **III)** in Gegenwart von Titantetrachlorid zum Enamin der Formel **IV**

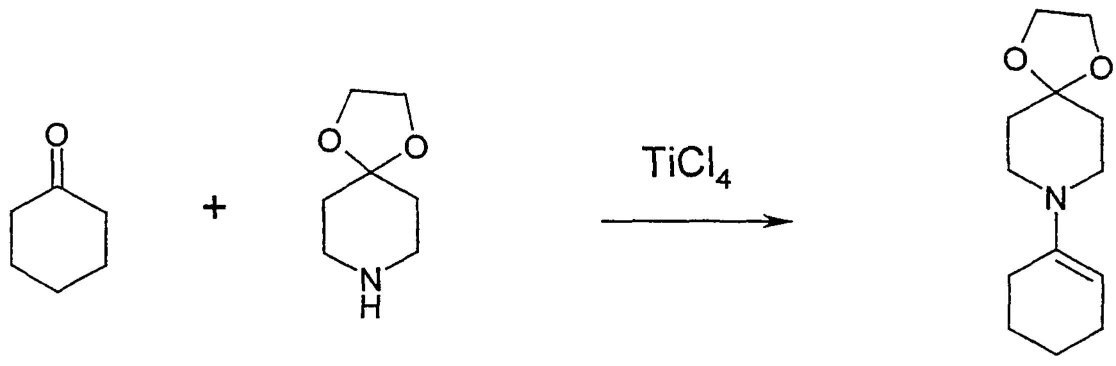


II III IV

umsetzt, welches weiter mit einem Phenylmagnesium-halogenid (Formel **V)** in Gegenwart von Trimethylchlorsilan zum Amin der Formel **VI**

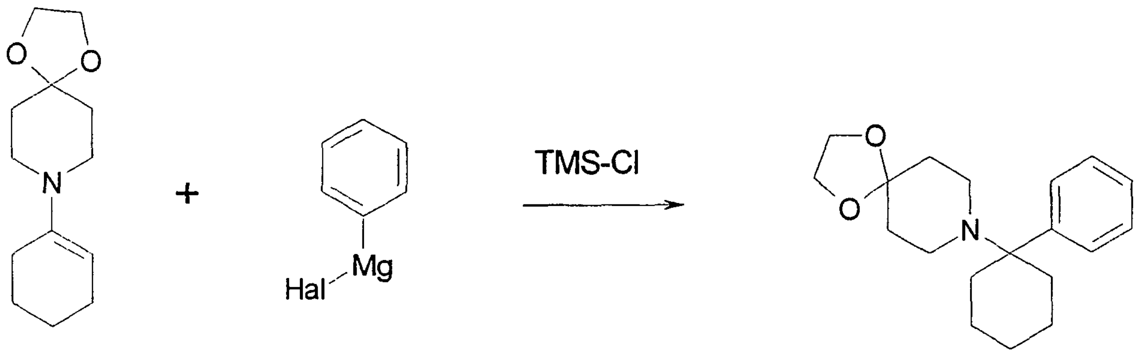


IV V VI

umgesetzt wird, anschließend das erhaltene Amin der Formel **VI** hydrolysiert und als Hydrochlorid der Formel **VII**

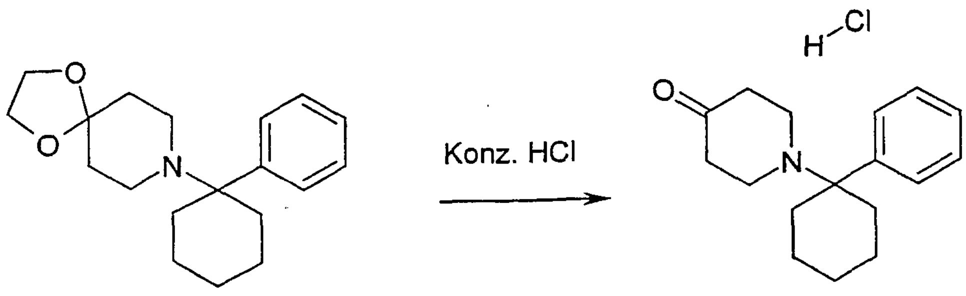


VI VII

in die freie Base der Formel **VIII** umgesetzt wird, wonach die Umsetzung mit einem Grignardreagenz **IX** zu den Verbindungen **X**

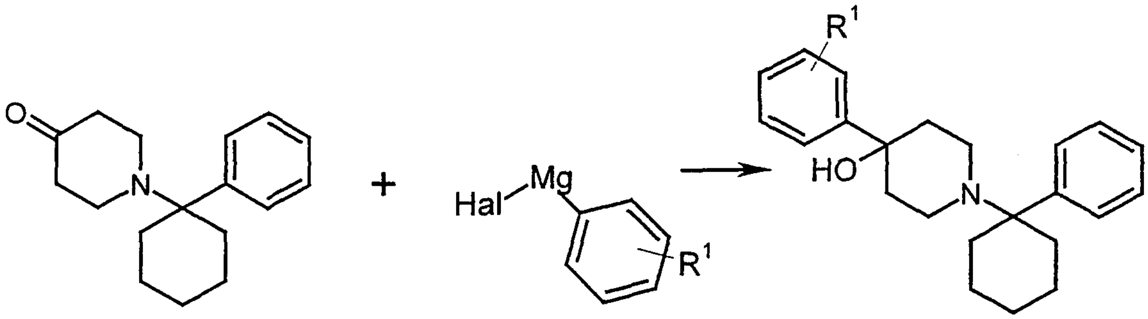

VIII IX X

erfolgt, die nach Fällung als Hydrochlorid **XI**

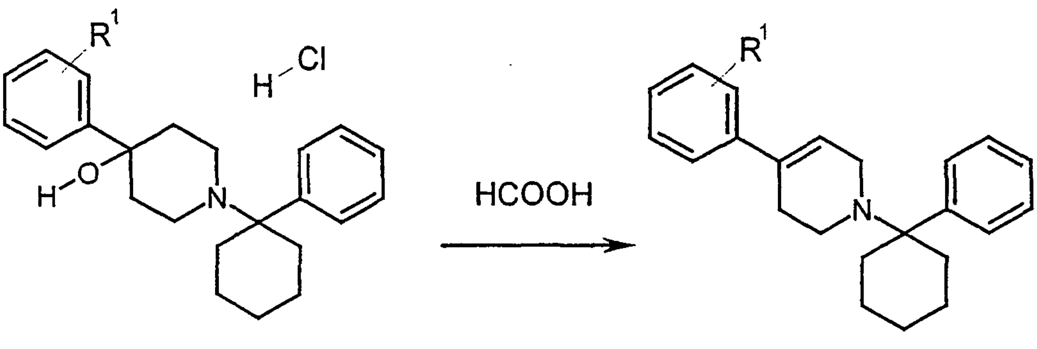


XI I

weiter mit Ameisensäure zu den Verbindungen der allgemeinen Formel **I** umgesetzt werden, die nach üblichen Methoden gereinigt und als Salze von physiologisch verträglichen Säuren isoliert werden.

**5.** Verfahren zur Herstellung einer Verbindung der Formel I nach Anspruch 4, **dadurch gekennzeichnet, daß** im Schritt 2, der Grignard-Reaktion, das Enamin (Formel **IV)** direkt in Gegenwart von etherischer HCl weiter mit Phenylmagnesium-halogenid (Formel **V**) zum Amin der Formel **VI** umgesetzt wird, wobei das Phenylmagnesium-halogenid (Formel **V**) vorgelegt wird.

6. Verfahren zur Herstellung einer Verbindung der Formel I nach Anspruch 4, **dadurch gekennzeichnet, daß** man Cylohexanon (Formel **II)** mit 1,4-Dioxa-8-aza-spiro [4.5]decane (Formel **III)** in Gegenwart von Kaliumcyanid und Salzsäure zum Aminonitril der Formel **XII** umsetzt,

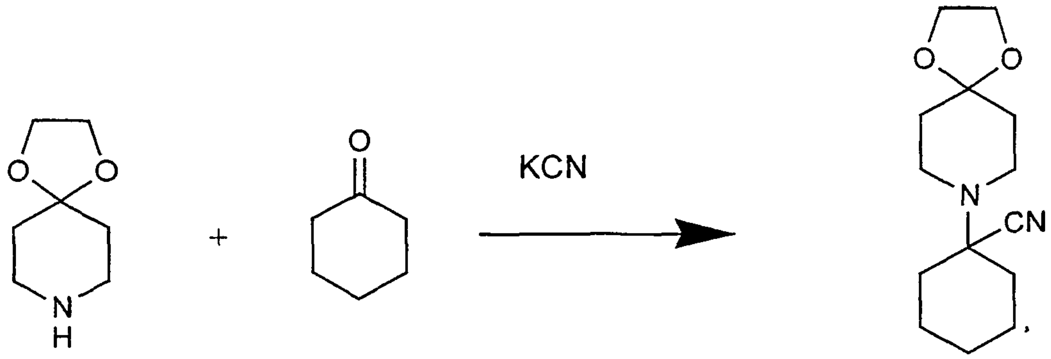


III II XII

das weiter mit Phenyl-Grignard-Reagenzien zum Amin der Formel **VI** umgesetzt wird,

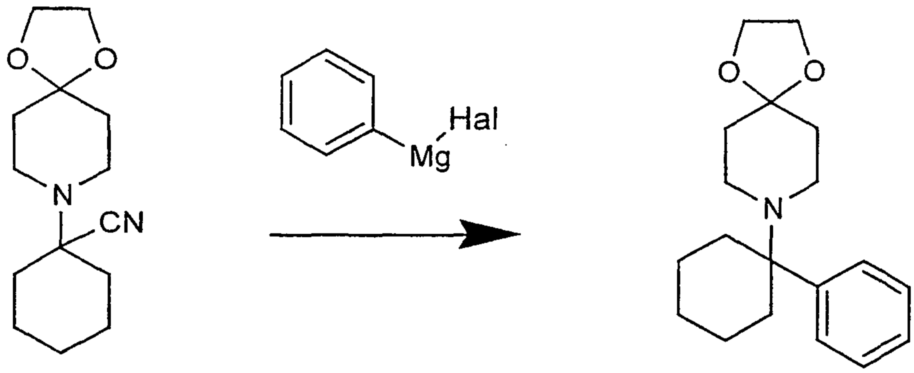

XII VI

und die nachfolgenden Verfahrensschritte wie oben beschrieben durchgeführt werden.

7. Verfahren zur Herstellung von substituierten 4-Phenyl-1-(1-phenyl-cyclohexyl)-1,2,3,6-tetrapyridinen der allgemeinen Formel I nach Anspruch 1, wobei man *N-tert.* Butyloxycarbonyl-4-piperidon (Formel **XIII)** und Grignard-Reagenz (Formel **IX)** zur Verbindung der Formel **XIV** umsetzt,

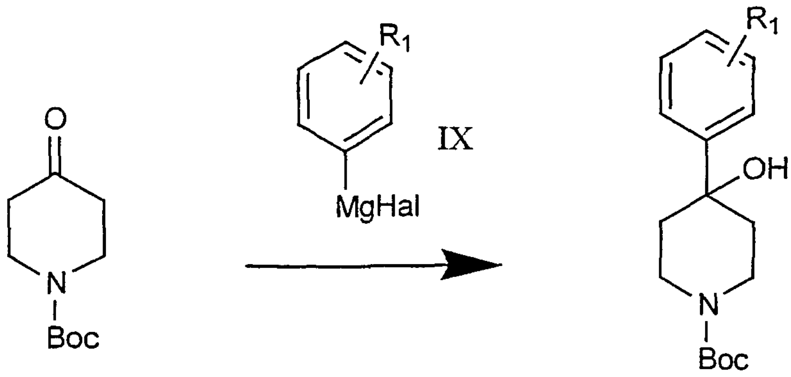


XIII XIV

aus welcher mit einer Säure, vorzugsweise HCl, HBr oder HBr/Eisessig oder Ameisensäure, die *tert.*-Butyloxycarbonyl-Schutzgruppe abgespalten, Wasser eliminiert und die Verbindung **XV** als HCl-Salz erhalten wird,

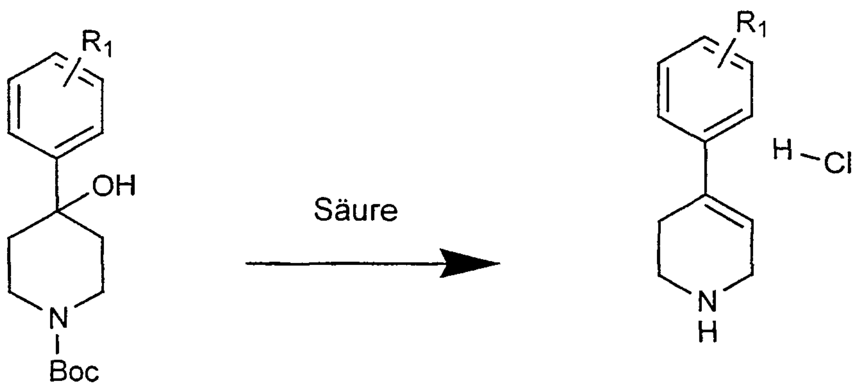


XIV XV

die weiter mit Titan(IV)chlorid und Cyclohexanon (Formel **II**) zu dem entsprechenden Enamin der Formel **XVI** umgesetzt wird,

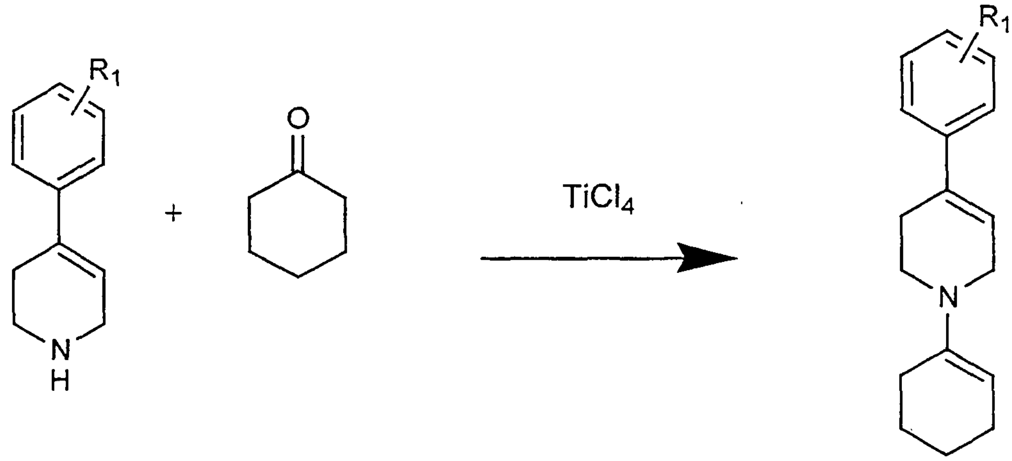


XV II XVI

welches in Gegenwart von Trimethylchlorsilan oder etherischer HCl mit Phenylmagnesium-halogenid umgesetzt wird,

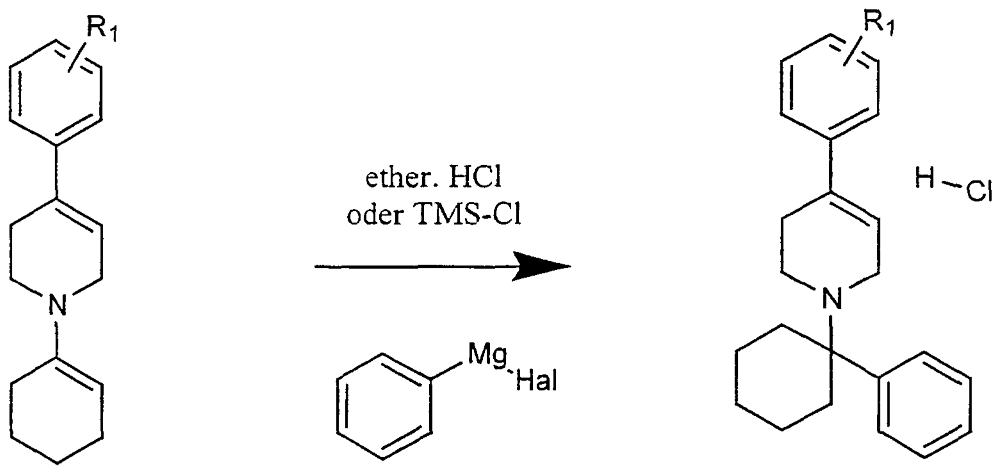


XVI I

und anschließend das HCl-Salz der entstandenen Verbindung der allgemeinen Formel **I** gefällt wird.

8. Arzneimittel enthaltend als Wirkstoff wenigstens eine Verbindung der allgemeinen Formel I gemäß Anspruch 1 und/oder deren Salze von physiologisch verträglichen Säuren.

9. Verwendung wenigstens einer Verbindung der allgemeinen Formel I nach Anspruch 1 und/oder von deren Salzen von physiologisch verträglichen Säuren zur Herstellung eines Arzneimittels zur Bekämpfung von Schmerzen.

10. Verwendung wenigstens einer Verbindung der allgemeinen Formel I nach Anspruch 1 und/oder von deren Salzen von physiologisch verträglichen Säuren zur Herstellung eines Arzneimittels zur Behandung von Epilepsie und/oder Schizophrenie.

11. Verwendung wenigstens einer Verbindung der allgemeinen Formel I nach Anspruch 1 und/oder von deren Salzen von physiologisch verträglichen Säuren zur Herstellung eines Arzneimittels zur Behandung von neurodegenerativen Erkrankungen.

12. Verwendung wenigstens einer Verbindung der allgemeinen Formel I nach Anspruch 1 und/oder von deren Salzen von physiologisch verträglichen Säuren zur Herstellung eines Arzneimittels zur Behandlung von cerebralen Ischämien.

13. Verwendung wenigstens einer Verbindung der allgemeinen Formel I nach Anspruch 1 und/oder von deren Salzen von physiologisch verträglichen Säuren zur Herstellung eines Arzneimittels zur Behandlung von cerebralen Infarkten.

14. Verwendung wenigstens einer Verbindung der allgemeinen Formel I nach Anspruch 1 und/oder von deren Salzen von physiologisch verträglichen Säuren zur Herstellung eines Arzneimittels zur Behandlung von Psychosen bedingt durch erhöhten Aminosäurespiegel.

15. Verwendung wenigstens einer Verbindung der allgemeinen Formel I nach Anspruch 1 und/oder von deren Salzen von physiologisch verträglichen Säuren zur Herstellung eines Arzneimittels zur Behandlung von Hirnödemen.

16. Verwendung wenigstens einer Verbindung der allgemeinen Formel I nach Anspruch 1 und/oder von deren Salzen von physiologisch verträglichen Säuren zur Herstellung eines Arzneimittels zur Behandlung von Unterversorgungszuständen des zentralen Nervensystems.

17. Verwendung wenigstens einer Verbindung der allgemeinen Formel I nach Anspruch 1 und/oder von deren Salzen von physiologisch verträglichen Säuren zur Herstellung eines Arzneimittels zur Behandlung von AIDS-Demenz.

18. Verwendung wenigstens einer Verbindung der allgemeinen Formel I nach Anspruch 1 und/oder von deren Salzen von physiologisch verträglichen Säuren zur Herstellung eines Arzneimittels zur Behandlung von Encephalomyelitis.

19. Verwendung wenigstens einer Verbindung der allgemeinen Formel I nach Anspruch 1 und/oder von deren Salzen von physiologisch verträglichen Säuren zur Herstellung eines Arzneimittels zur Behandlung des Tourette-Syndroms.

20. Verwendung wenigstens einer Verbindung der allgemeinen Formel I nach Anspruch 1 und/oder von deren Salzen von physiologisch verträglichen Säuren zur Herstellung eines Arzneimittels zur Behandlung perinataler Asphyxie.

21. Verwendung wenigstens einer Verbindung der allgemeinen Formel I nach Anspruch 1 und/oder von deren Salzen von physiologisch verträglichen Säuren zur Herstellung eines Arzneimittels zur Behandlung von Tinnitus.

22. Verwendung wenigstens einer Verbindung der allgemeinen Formel I nach Anspruch 1 und/oder von deren Salzen von physiologisch verträglichen Säuren zur Herstellung eines Arzneimittels zur Prophylaxe von Schlaganfällen.

## Claims

1. Substituted 4-phenyl-1-(1-phenylcyclohexyl)-1,2,3,6-tetrahydropyridines of the general formula I,

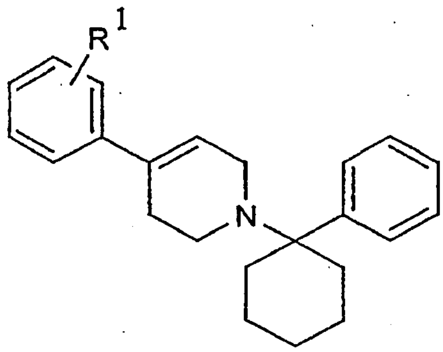

I

wherein $R^1$ denotes $C_1$-$C_5$-alkyl (branched or unbranched), halogen (except 4-Cl) , O-$C_1$-$C_5$-alkyl (branched or unbranched), S-$C_1$-$C_5$-alkyl (branched or unbranched), and/or their salts of physiologically compatible acids.

**2.** Compounds according to claim 1, **characterised in that** $R^1$ denotes methyl, chlorine (except in the 4-position), fluorine, methoxy or methylsulfanyl.

**3.** Compounds according to claim 1:

4-(2-fluorophenyl)-1-(1-phenylcyclohexyl)-1,2,3,6-tetrahydropyridine or the corresponding hydrochloride
4-(3-fluorophenyl)-1-(1-phenylcyclohexyl)-1,2,3,6-tetrahydropyridine or the corresponding hydrochloride
9-(4-fluorophenyl)-1-(1-phenylcyclohexyl)-1,2,3,6-tetrahydropyridine or the corresponding hydrochloride
4-(2-methoxyphenyl)-1-(1-phenylcyclohexyl)-1,2,3,6-tetrahydropyridine or the corresponding hydrochloride
4-(3-methoxyphenyl)-1-(1-phenylcyclohexyl)-1,2,3,6-tetrahydropyridine or the corresponding hydrochloride
4-(4-methoxyphenyl)-1-(1-phenylcyclohexyl)-1,2,3,6-tetrahydropyridine or the corresponding hydrochloride
4-(2-methylphenyl)-1-(1-phenylcyclohexyl)-1,2,3,6-tetrahydropyridine or the corresponding hydrochloride
4-(3-methylphenyl)-1-(1-phenylcyclohexyl)-1,2,3,6-tetrahydropyridine or the corresponding hydrochloride
4-(4-methylphenyl)-1-(1-phenylcyclohexyl)-1,2,3,6-tetrahydropyridine or the corresponding hydrochloride
4-(2-methylsulfanylphenyl)-1-(1-phenylcyclohexyl)-1,2,3,6-tetrahydropyridine or the corresponding hydrochloride
4-(2-chlorophenyl)-1-(l-phenylcyclohexyl)-1,2,3,6-tetrahydropyridine or the corresponding hydrochloride.

**4.** Process for the production of substituted 4-phenyl-1-(1-phenylcyclohexyl)-1,2,3,6-tetrahydropyridines of the general formula I according to claim 1, wherein cyclohexanone (formula **II**) is reacted with 1,4-dioxa-8-aza-spiro[4,5]decane (formula **III)** in the presence of titanium tetrachloride to form the enamine of the formula **IV**

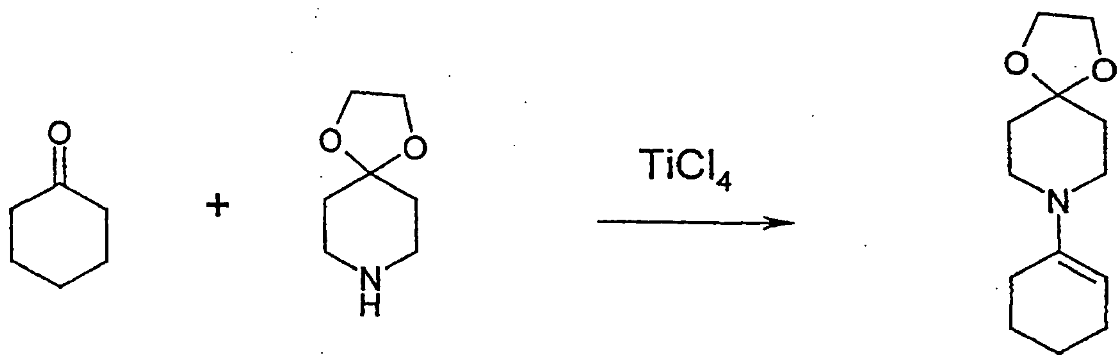


II III IV

which is then reacted further with a phenylmagnesium halide (formula **V**) in the presence of trimethylchlorosilane

to form the amine of the formula **VI**

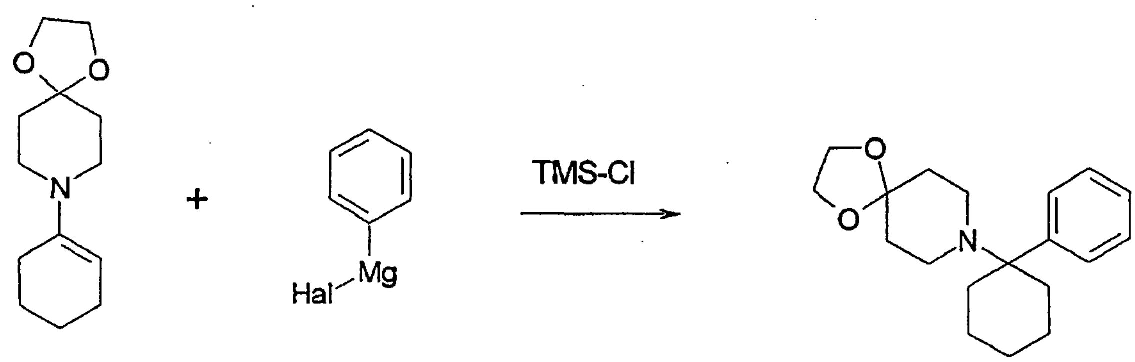


IV V VI

the amine obtained of the formula **VI** is then hydrolysed and as hydrochloride of the formula **VII**

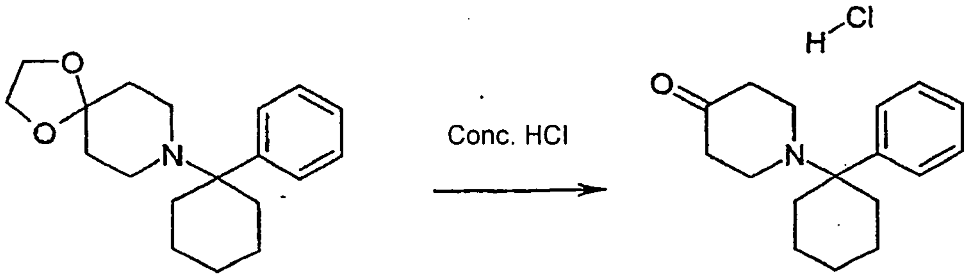


VI VII

is converted into the free base of the formula **VIII**, following which it is reacted with a Grignard reagent **IX** to form the compounds **X**

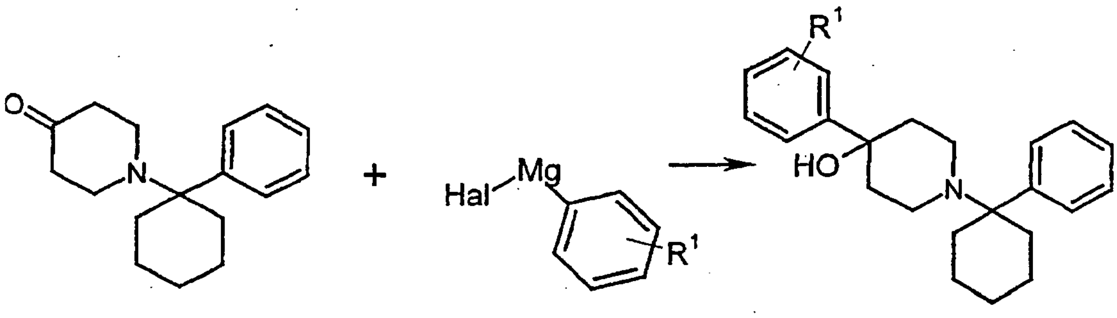

VIII IX X

which after precipitation as hydrochloride **XI**

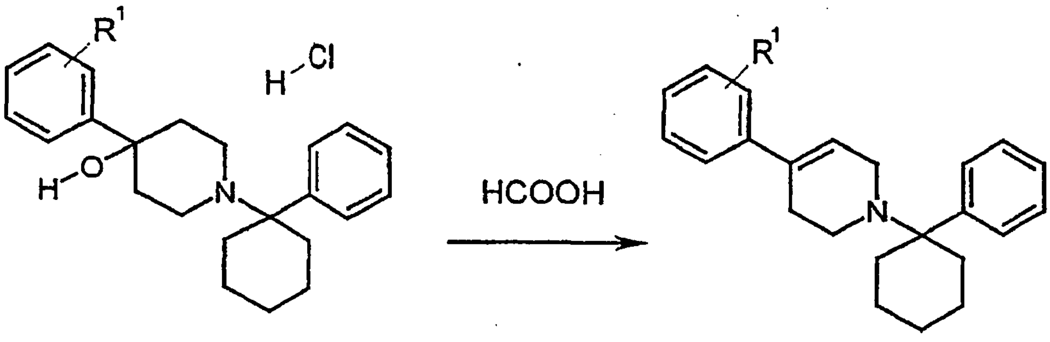


XI I

are reacted further with formic acid to form the compounds of the general formula I, which are purified by conventional methods and are isolated as salts of physiologically compatible acids.

**5.** Process for the production of a compound of the formula I according to claim 4, **characterised in that** in the 2nd stage, i.e. the Grignard reaction, the enamine (formula **IV)** is directly reacted further in the presence of ethereal HCl with phenylmagnesium halide (formula **V)** that was previously introduced, to form the amine of the formula **VI.**

**6.** Process for the production of a compound of the formula **I** according to claim 4, **characterised in that** cyclohexanone (formula **II)** is reacted with 1,4-dioxa-8-aza-spiro[4.5]decane (formula **III)** in the presence of potassium cyanide and hydrochloric acid to form the aminonitrile of the formula **XII,**

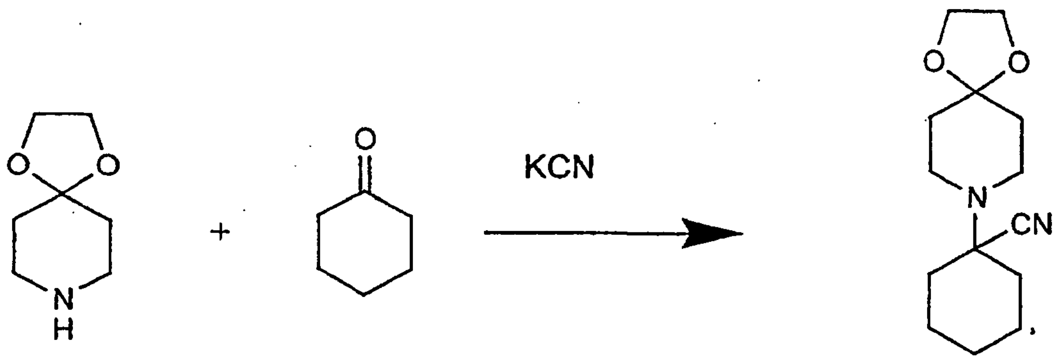


III II XII

which is further reacted with phenyl Grignard reagents to form the amine of the formula **VI**,

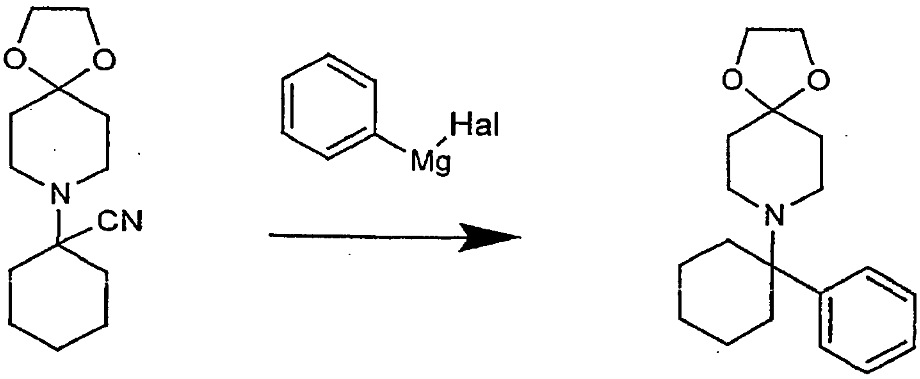

XII VI

and the subsequent process stages are as described above.

**7.** Process for the production of substituted 4-phenyl-1-(1-phenylcyclohexyl)-1,2,3,6-tetrapyridines of the general formula I according to claim 1, in which **N-**tert.-butyloxycarbonyl-4-piperidone (formula **XIII)** and Grignard reagent (formula **IX)** are reacted to form the compound of the formula **XIV**

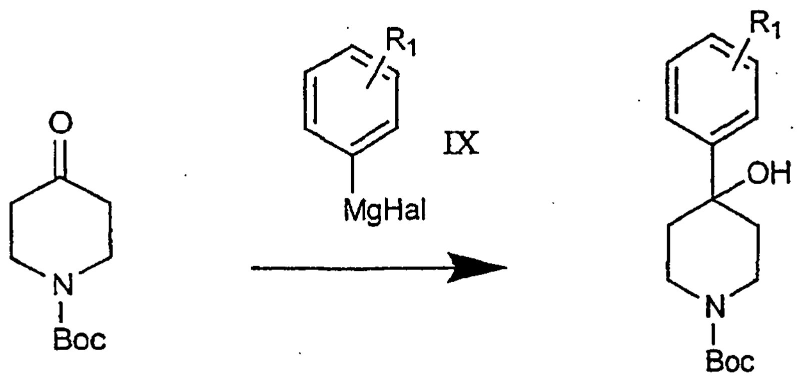

IX

XIII XIV

from which the tert.-butyloxycarbonyl protective group is split off with an acid, preferably HCl, HBr or HBr/glacial acetic acid or formic acid, with the elimination of water, and the compound **XV** is obtained as the HCl salt,

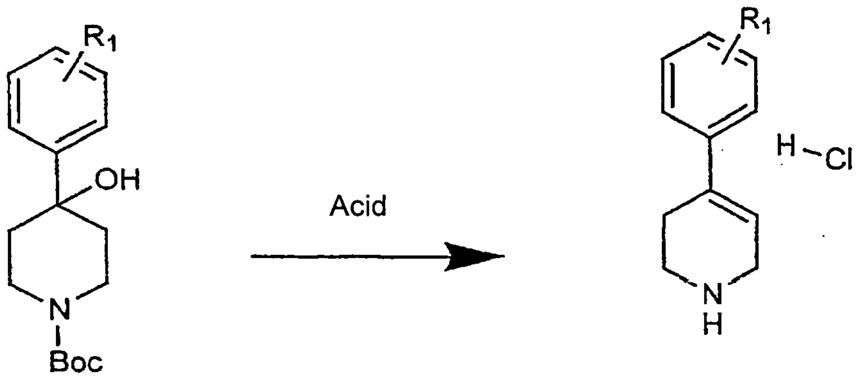


XIV XV

which is reacted further with titanium(IV) chloride and cyclohexanone (formula **II**) to form the corresponding enamine of the formula **XVI,**

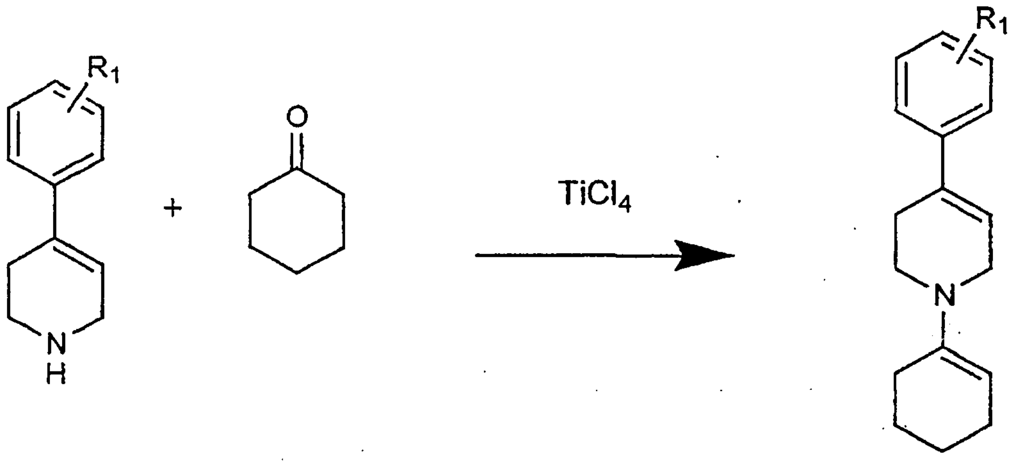


XV II XVI

which is reacted with phenylmagnesium halide in the presence of trimethylchlorosilane or ethereal HCl,

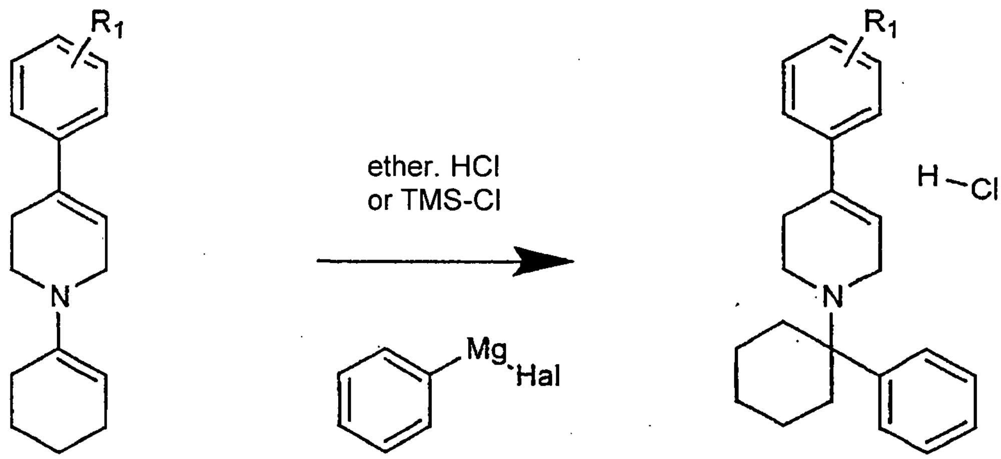


XVI I

and the HCl salt of the compound formed of the general formula **I** is then precipitated.

**8.** Medicament containing as active substance at least one compound of the general formula I according to claim 1 and/or their salts of physiologically compatible acids.

**9.** Use of at least one compound of the general formula I according to claim 1 and/or of their salts of physiologically compatible acids to produce a medicament to relieve pain.

**10.** Use of at least one compound of the general formula I according to claim 1 and/or of their salts of physiologically compatible acids to produce a medicament for the treatment of epilepsy and/or schizophrenia.

**11.** Use of at least one compound of the general formula I according to claim 1 and/or of their salts of physiologically compatible acids to produce a medicament for the treatment of neurodegenerative diseases.

**12.** Use of at least one compound of the general formula I according to claim 1 and/or of their salts of physiologically compatible acids to produce a medicament for the treatment of cerebral ischaemias.

**13.** Use of at least one compound of the general formula I according to claim 1 and/or of their salts of physiologically compatible acids to produce a medicament for the treatment of cerebral infarcts.

**14.** Use of at least one compound of the general formula I according to claim 1 and/or of their salts of physiologically compatible acids to produce a medicament for the treatment of psychoses due to raised amino acid levels.

**15.** Use of at least one compound of the general formula I according to claim 1 and/or of their salts of physiologically compatible acids to produce a medicament for the treatment of brain swellings.

**16.** Use of at least one compound of the general formula I according to claim 1 and/or of their salts of physiologically compatible acids to produce a medicament for the treatment of deficiency states of the central nervous system.

**17.** Use of at least one compound of the general formula I according to claim 1 and/or of their salts of physiologically compatible acids to produce a medicament for the treatment of AIDS dementia.

**18.** Use of at least one compound of the general formula I according to claim 1 and/or of their salts of physiologically compatible acids to produce a medicament for the treatment of encephalomyelitis.

**19.** Use of at least one compound of the general formula I according to claim 1 and/or of their salts of physiologically compatible acids to produce a medicament for the treatment of Tourette's syndrome.

**20.** Use of at least one compound of the general formula I according to claim 1 and/or of their salts of physiologically compatible acids to produce a medicament for the treatment of perinatal asphyxia.

**21.** Use of at least one compound of the general formula I according to claim 1 and/or of their salts of physiologically compatible acids to produce a medicament for the treatment of tinnitus.

**22.** Use of at least one compound of the general formula I according to claim 1 and/or of their salts of physiologically compatible acids to produce a medicament for the prophylaxis of strokes.

## Revendications

**1.** 4-phényl-1-(1-phényl-cyclohexyl)-1,2,3,6-tétrahydropyridines substituées de formule générale I,

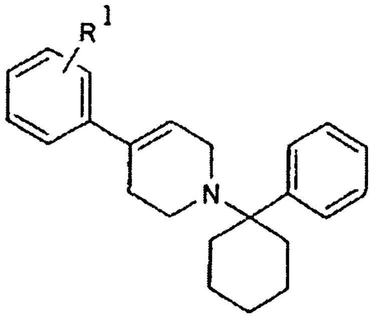

I

dans laquelle $R^1$ est un reste alkyle en $C_1$ à $C_5$ (ramifié ou non ramifié), un halogène (excepté 4-Cl), un reste O-alkyle en $C_1$ à $C_5$ (ramifié ou non ramifié), un reste S-alkyle en $C_1$ à $C_5$ (ramifié ou non ramifié) et/ou leurs sels d'acides physiologiquement acceptables.

**2.** Composés suivant la revendication 1, **caractérisés en ce que** $R^1$ représente le reste méthyle, le chlore (excepté en position 4), le fluor, le reste méthoxy ou le reste méthylsulfanyle.

**3.** Composés suivant la revendication 1 :

4-(2-fluorophényl)-1-(1-phényl-cyclohexyl)-1,2,3,6-tétrahydropyridine ou le chlorhydrate correspondant
4-(3-fluorophényl)-1-(1-phényl-cyclohexyl)-1,2,3,6-tétrahydropyridine ou le chlorhydrate correspondant
4-(4-fluorophényl)-1-(1-phényl-cyclohexyl)-1,2,3,6-tétrahydropyridine ou le chlorhydrate correspondant
4-(2-méthoxyphényl)-1-(1-phényl-cyclohexyl)-1,2,3,6-tétrahydropyridine ou le chlorhydrate correspondant
4-(3-méthoxyphényl)-1-(1-phényl-cyclohexyl)-1,2,3,6-tétrahydropyridine ou le chlorhydrate correspondant
4-(4-méthoxyphényl)-1-(1-phényl-cyclohexyl)-1,2,3,6-tétrahydropyridine ou le chlorhydrate correspondant
4-(2-méthylphényl)-1-(1-phényl-cyclohexyl)-1,2,3,6-tétrahydropyridine ou le chlorhydrate correspondant
4-(3-méthylphényl)-1-(1-phényl-cyclohexyl)-1,2,3,6-tétrahydropyridine ou le chlorhydrate correspondant
4-(4-méthylphényl)-1-(1-phényl-cyclohexyl)-1,2,3,6-tétrahydropyridine ou le chlorhydrate correspondant
4-(2-méthylsulfanyl-phényl)-1-(1-phényl-cyclohexyl)-1,2,3,6-tétra-hydropyridine ou le chlorhydrate correspondant
4-(2-chlorophényl)-1-(1-phényl-cyclohexyl)-1,2,3,6-tétrahydropyridine ou le chlorhydrate correspondant.

**4.** Procédé de production de 4-phényl-1-(1-phényl-cyclohexyl)-1,2,3,6-tétrahydropyridines substituées de formule générale I suivant la revendication 1, dans lequel on fait réagir la cyclohexanone (formule II) avec le 1,4-dioxa-8-aza-spiro [4.5] décane (formule III) en présence de tétrachlorure de titane pour former l'énamine de formule IV,

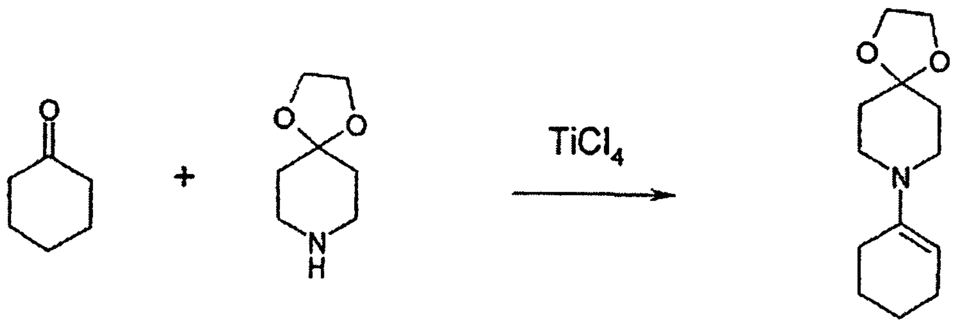


II III IV

qu'on fait ensuite réagir avec un halogénure de phénylmagnésium (formule V) en présence de triméthylchlorosilane pour former l'amine de formule VI

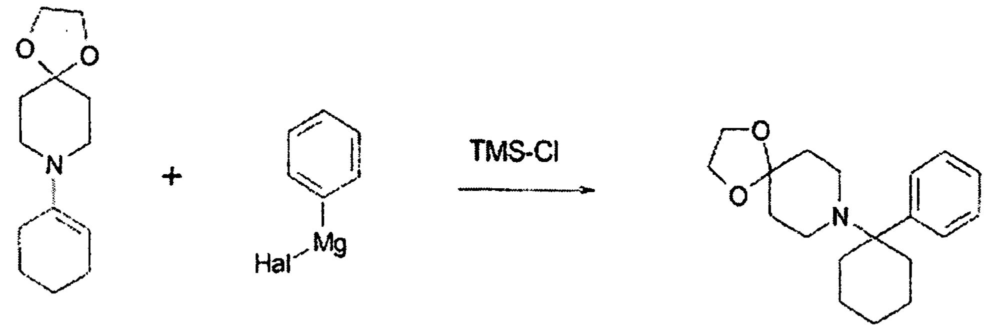


IV V VI

l'amine obtenue de formule VI est ensuite hydrolysée et, à l'état de chlorhydrate de formule VII

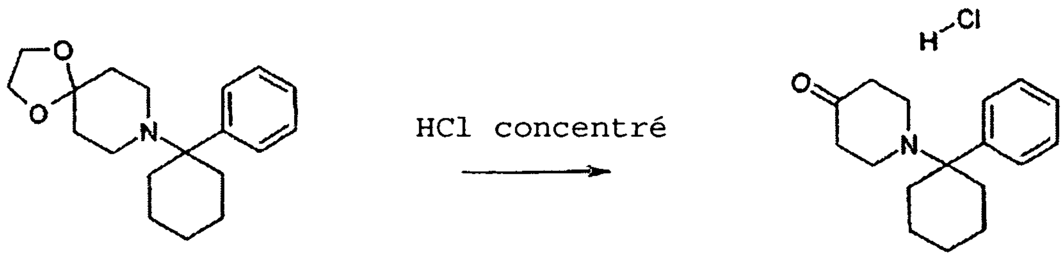


VI VII

elle est transformée en la base libre de formule VIII, qui est ensuite amenée à réagir avec un réactif de Grignard IX pour former les composés X

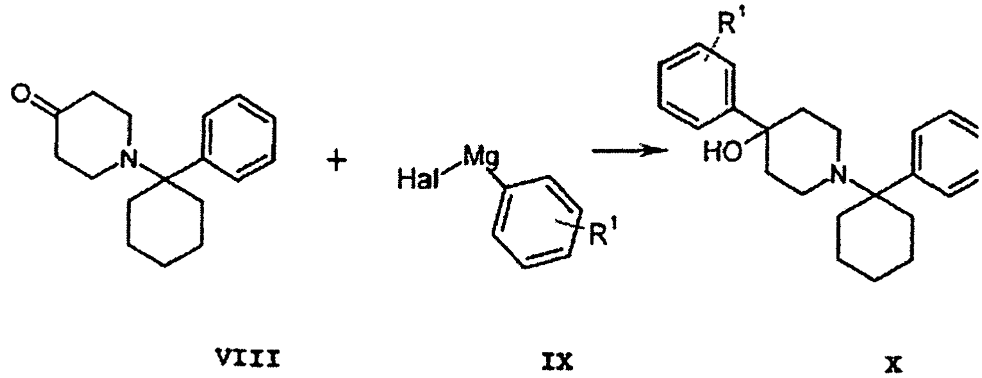


qui, après précipitation sous forme de chlorhydrate XI, sont ensuite transformés par réaction avec l'acide formique en composés de formule générale I

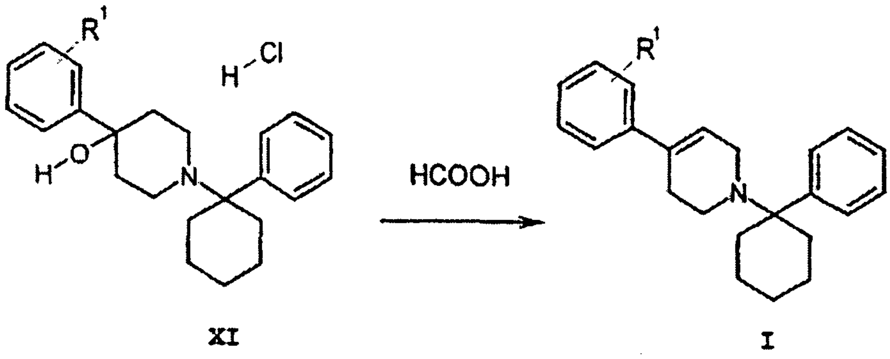


qui sont purifiés par des opérations usuelles et isolés sous forme de sels d'acides physiologiquement acceptables.

5. Procédé de production d'un composé de formule I suivant la revendication 4, **caractérisé en ce que** dans la deuxième étape ou réaction de Grignard, l'énamine (formule IV) est amenée à réagir directement en présence de HCl dans l'éther avec un halogénure de phénylmagnésium (formule V) pour former l'amine VI, l'halogénure de phénylmagnésium (formule V) étant introduit au début.

6. Procédé de production d'un composé de formule I suivant la revendication 4, **caractérisé en ce qu'**on fait réagir la cyclohexanone (formule II) avec le 1,4-dioxa-8-aza-spiro[4,5]décane (formule III) en présence de cyanure de potassium et d'acide chlorhydrique pour obtenir l'aminonitrile de formule **XII**

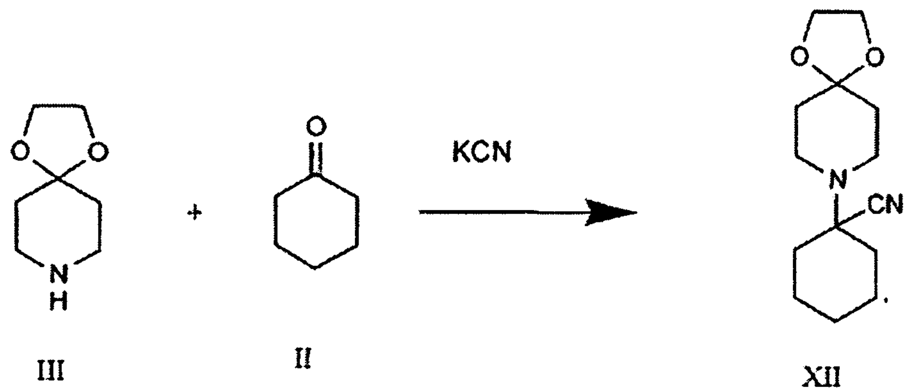

qu’on fait ensuite réagir avec des réactifs phényliques de Grignard pour obtenir l’amine de formule VI

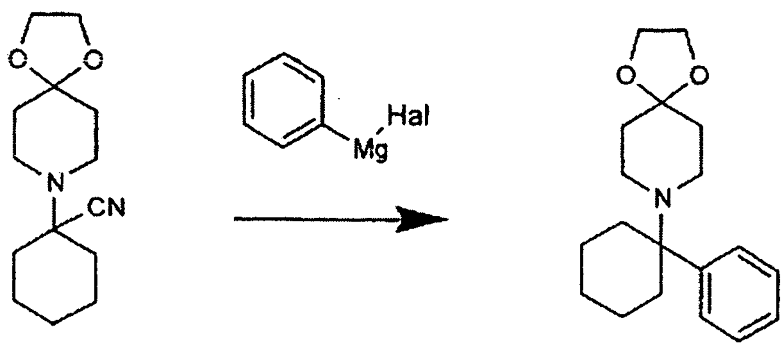

XII VI

les étapes subséquentes du procédé étant conduites de la manière décrite ci-dessus.

**7.** Procédé de production de 4-phényl-1-(1-phényl-cyclohexyl)-1,2,3,6-tétrahydropyridines substituées de formule générale I suivant la revendication 1, dans lequel on fait réagir la *N*-tertiobutyloxycarbonyl-4-pipéridone (formule XIII) et un réactif de Grignard (formule IX) pour obtenir le composé de formule XIV,

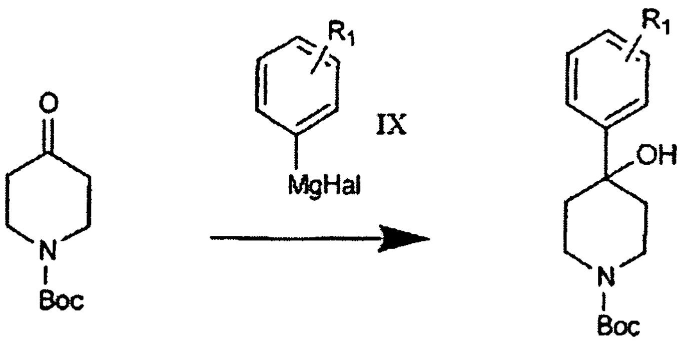

IX

XIII XIV

duquel on élimine le groupe protecteur tertiobutyloxy-carbonyle avec un acide, avantageusement HCl, HBr ou HBr/acide acétique cristallisable ou l’acide formique, on élimine l’eau et on obtient le composé XV sous forme du sel de HCl,

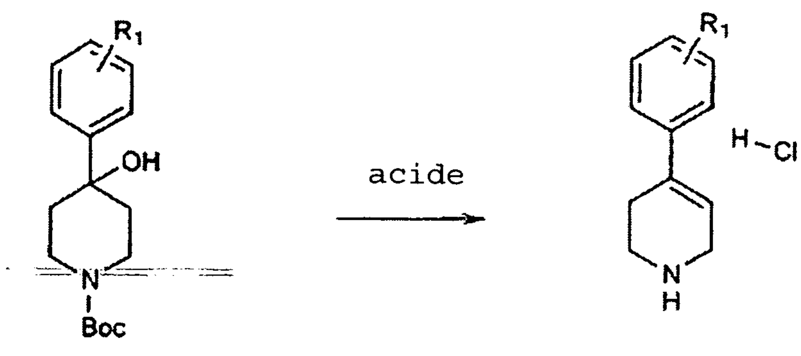


XIV XV

qu'on fait ensuite réagir avec le chlorure de titane (IV) et la cyclohexanone (formule II) pour obtenir l'énamine correspondante de formule XVI,

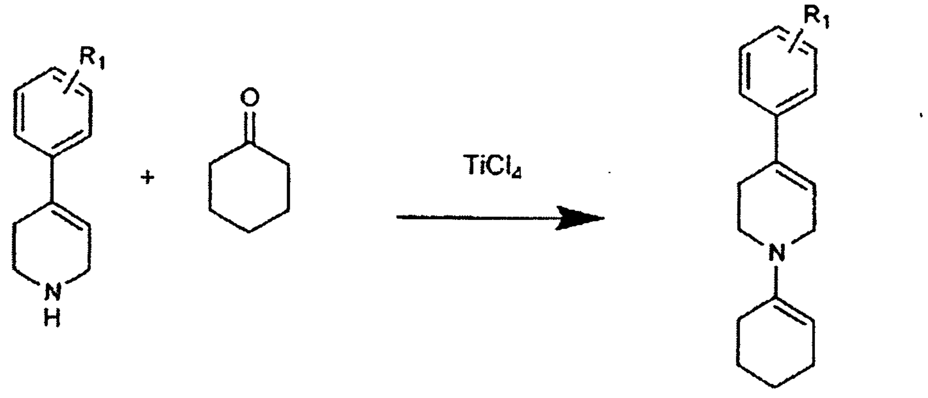


XV II XVI

qu'on fait réagir en présence de triméthylchlorosilane ou de HCl dans l'éther avec un halogénure de phénylmagnésium,

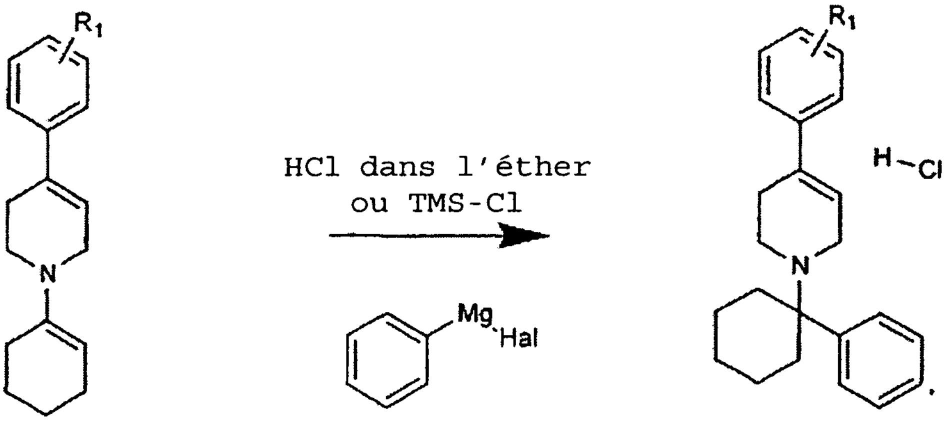


XVI I

puis on précipite le chlorhydrate du composé de formule générale I qui est produit.

**8.** Médicament contenant, comme substance active, au moins un composé de formule générale I suivant la revendication 1 et/ou ses sels d'acides physiologiquement acceptables.

**9.** Utilisation d'au moins un composé de formule générale I suivant la revendication 1 et/ou de ses sels d'acides physiologiquement acceptables pour la préparation d'un médicament destiné à combattre des douleurs.

**10.** Utilisation d'au moins un composé de formule générale I suivant la revendication 1 et/ou de ses sels d'acides physiologiquement acceptables pour la préparation d'un médicament destiné au traitement de l'épilepsie et/ou de la schizophrénie.

**11.** Utilisation d'au moins un composé de formule générale I suivant la revendication 1 et/ou de ses sels d'acides physiologiquement acceptables pour la préparation d'un médicament destiné au traitement de maladies neurodégénératives.

**12.** Utilisation d'au moins un composé de formule générale I suivant la revendication 1 et/ou de ses sels d'acides physiologiquement acceptables pour la préparation d'un médicament destiné au traitement d'ischémie cérébrale.

**13.** Utilisation d'au moins un composé de formule générale I suivant la revendication 1 et/ou de ses sels d'acides physiologiquement acceptables pour la préparation d'un médicament destiné au traitement d'infarctus cérébraux.

**14.** Utilisation d'au moins un composé de formule générale I suivant la revendication 1 et/ou de ses sels d'acides physiologiquement acceptables pour la préparation d'un médicament destiné au traitement de psychoses dues à des taux élevés d'aminoacides.

**15.** Utilisation d'au moins un composé de formule générale I suivant la revendication 1 et/ou de ses sels d'acides physiologiquement acceptables pour la préparation d'un médicament destiné au traitement d'oedèmes cérébraux.

**16.** Utilisation d'au moins un composé de formule générale I suivant la revendication 1 et/ou de ses sels d'acides physiologiquement acceptables pour la préparation d'un médicament destiné au traitement d'états d'approvisionnement déficitaire du système nerveux central.

**17.** Utilisation d'au moins un composé de formule générale I suivant la revendication 1 et/ou de ses sels d'acides physiologiquement acceptables pour la préparation d'un médicament destiné au traitement de la démence liée au SIDA.

**18.** Utilisation d'au moins un composé de formule générale I suivant la revendication 1 et/ou de ses sels d'acides physiologiquement acceptables pour la préparation d'un médicament destiné au traitement de l'encéphalomyélite.

**19.** Utilisation d'au moins un composé de formule générale I suivant la revendication 1 et/ou de ses sels d'acides physiologiquement acceptables pour la préparation d'un médicament destiné au traitement du syndrome de Tourette.

**20.** Utilisation d'au moins un composé de formule générale I suivant la revendication 1 et/ou de ses sels d'acides physiologiquement acceptables pour la préparation d'un médicament destiné au traitement de l'asphyxie périnatale.

**21.** Utilisation d'au moins un composé de formule générale I suivant la revendication 1 et/ou de ses sels d'acides physiologiquement acceptables pour la préparation d'un médicament destiné au traitement des acouphènes.

**22.** Utilisation d'au moins un composé de formule générale I suivant la revendication 1 et/ou de ses sels d'acides physiologiquement acceptables pour la préparation d'un médicament destiné au traitement à la prophylaxie d'apoplexies cérébrales.